# EUROPEAN PATENT APPLICATION

(11) **EP 4 578 878 A1**
(43) Date of publication of application: **02.07.2025**
(21) Application number: 23870950.5
(22) Date of filing: 27.09.2023
(51) Int. Cl.: C07K 19/00, C07K 14/135, C12N 15/62, C12N 15/85, C12N 5/10, A61K 39/155, A61P 31/14

(54) **RESPIRATORY SYNCYTIAL VIRUS RECOMBINANT FUSION PROTEIN HAVING PRE-FUSION CONFORMATION, AND PREPARATION METHOD THEREFOR AND USE THEREOF**

(30) Priority: 29.09.2022 CN 202211199073
(71) Applicant: Beijing Benewill Technology Development Co., Ltd., Beijing 102206 (CN)
(72) Inventor: YAN, Jinghua, Beijing 102206 (CN); HUANG, Qingrui, Beijing 102206 (CN); YANG, Mi, Beijing 102206 (CN)
(74) Representative: LLR
(86) International application number: PCT/CN2023/122155
(87) International publication number: WO 2024/067723

(57) **Abstract**

The present application relates to a respiratory syncytial virus subtype A (RSV-A) recombinant F protein, a polynucleotide encoding the RSV-A recombinant F protein, a nucleic acid construct comprising the polynucleotide, an expression vector comprising the nucleic acid construct, a host cell into which the polynucleotide, the nucleic acid construct, or the expression vector is transformed or transfected, a stabilized trimer formed from the RSV-A recombinant F protein, an immunogenic composition comprising any of the foregoing, and use of any of the forgoing in the preparation of a vaccine for the prevention and/or treatment of respiratory syncytial virus infections. The RSV-A recombinant F protein of the present application comprises at least one epitope specific to the pre-fusion F protein, and can form a stable, pre-fusion conformation of F protein trimer. Furthermore, the RSV-A recombinant F protein of the present application can be expressed stably in a uniform form and with a significantly increased yield. The formed F protein trimer has strong immunogenicity and thus can stimulate the body to produce a high-level antibody titer, which is of great significance for the clinical treatment and prevention of respiratory syncytial virus.

## Description

### Cross-Reference to Related Applications

The present application claims priority to Chinese patent application No. 202211199073.9 filed on September 29, 2022 and entitled "RESPIRATORY SYNCYTIAL VIRUS RECOMBINANT FUSION PROTEIN WITH PR-EFUSION CONFORMATION, PREPARATION METHOD AND USES THEREOF", which is incorporated herein by reference in its entirety.

### Technical Field

The present application relates to the technical field of vaccines, in particular to a respiratory syncytial virus subtype A (RSV-A) recombinant F protein, a stabilized trimer formed from the same, an immunogenic composition comprising the same, and a method for preparing the same and use thereof.

### Background Art

Respiratory syncytial virus (RSV) is the most common pathogen causing acute respiratory infections in infants. Bronchiolitis resulting from RSV infection is one of the leading causes of hospitalization of children under 2 years old, and a significant contributor to infant mortality. According to WHO estimates, approximately 64 million children worldwide are infected with RSV every year, of which 160,000 children die from RSV infections. In 2020, the number of severe RSV infections among children under 5 years old reached 34.6 million globally, with 3 million cases reported in China alone. Another high-risk group for RSV is the elderly. Several prospective studies have documented the incidence of RSV in community-dwelling older adults, revealing an annual infection rate between 2% and 10%. Large-scale epidemiological studies have revealed that RSV causes hospitalization and mortality rates comparable to those of influenza in the elderly population.

RSV has only one single serotype with two major antigenic subtypes, A and B. Strains of both subtypes usually co-circulate, but only one of them usually dominates during epidemic periods. In temperate regions, including China, RSV infections exhibit strong seasonality, typically emerging in late autumn to early winter, peaking from mid-December to early February, and declining in late spring. There is an urgent need worldwide for safe, effective, and affordable methods to prevent and treat RSV infections.

RSV, belonging to the genus *Pneumovirus* within the family *Paramyxoviridae,* is a pleomorphic, enveloped virus with a diameter of approximately 120-300 nm and has a non-segmented, negative-sense, single-stranded RNA genome (15-16 kb), which encodes 11 proteins. The viral envelope contains three proteins, namely the attachment (G) glycoprotein, the fusion (F) glycoprotein, and the small hydrophobic (SH) protein. The G protein plays a role in host cell attachment and the F protein is responsible for fusion and cell entry, whereas the SH protein is not required for either process. Two surface glycoproteins of RSV are the key neutralizing antigens: the G protein has high sequence diversity and determines the antigenic subtypes of the virus (A and B), whereas the F protein is highly conserved across both subtypes and is recognized by a broad range of cross-neutralizing antibodies. Due to the critical role in RSV invasion and its highly conserved protein sequence, the RSV F protein is the primary target of neutralizing antibodies and a major antigen for vaccine development. The F protein, a type I transmembrane protein, is initially synthesized as a 574-amino acid precursor protein F0 containing 5 to 6 post-translational N-linked glycosylations. To become functionally active, F0 undergoes proteolytic cleavage by a cellular furin-like protease at two multi-basic sites in the trans-Golgi network to produce the F1 (amino acids 137-574) and F2 (amino acids 26-109) subunits, along with the Pep27 polypeptide. The final functional form consists of the F1 and F2 subunits linked by a disulfide bond, with molecular weights of 55kDa and 15kDa, respectively. The homologous F protein trimer comprises both pre-fusion and post-fusion conformations. The pre-fusion F protein is a metastable structure, and transforms into the stable post-fusion F protein upon viral-cell fusion, a process that can also occur spontaneously. Since the epitopes of highly potent neutralizing antibodies against the F protein are primarily located on the pre-fusion F protein, how to maintain the F protein in the pre-fusion conformation is a critical challenge in the development of RSV vaccines. To date, no RSV vaccine has been approved for market use.

### Summary of the Invention

To overcome the defects present in the prior art, provided herein is a respiratory syncytial virus subtype A (RSV-A) recombinant F protein ((herein referred to as "RSV-A-Fm" or "Fm"), a polynucleotide encoding the RSV-A recombinant F protein, a nucleic acid construct comprising the polynucleotide, an expression vector comprising the nucleic acid construct, a host cell into which the polynucleotide, the nucleic acid construct, or the expression vector is transformed or transfected, a stabilized trimer formed from the RSV-A recombinant F protein, an immunogenic composition comprising any of the foregoing, and the use thereof in the preparation of a vaccine for the prevention and/or treatment of respiratory syncytial virus infection.

Specifically, the invention provides the following technical solutions:
In a first aspect, the present application provides a respiratory syncytial virus subtype A (RSV-A) recombinant F protein (herein also referred to as RSV-A recombinant protein or RSV-A recombinant F protein), which is derived from respiratory syncytial virus subtype A. The RSV-A recombinant F protein comprises RSV-A-F₁ peptide fragment and RSV-A-F₂ peptide fragment, wherein the RSV-A-F₁ peptide fragment and/or the RSV-A-F₂ peptide fragment comprise at least one mutation to proline compared to the corresponding peptide fragment of the wild-type RSV-A F protein.

In one embodiment, the RSV-A-F₁ peptide fragment corresponds to the amino acid fragment at positions 26-99, 26-103, 26-108, or 26-97 in the amino acid sequence of the wild-type RSV-A F protein as set forth in SEQ ID NO: 1, the RSV-A-F₂ peptide fragment corresponds to the amino acid fragment at positions 136-513, 145-513, 138-513, or 137-513 in the amino acid sequence of the wild-type RSV-A F protein as set forth in SEQ ID NO: 1, and the RSV-A-F₁ peptide fragment and/or the RSV-A-F₂ peptide fragment comprise one or more mutations to proline compared to the corresponding peptide fragments of the wild-type RSV-A F protein.

Preferably, the one or more mutations to proline are selected from the following mutations in the amino acid sequence of the wild-type RSV-A F protein as set forth in SEQ ID NO: 1:
K65P, N67P, D73P, L138P, G139P, L141P, E161P, Q210P, I214P, S215P, N216P, Q279P, and S377P.

More preferably, the one or more mutations to proline are selected from the following mutations or combinations of mutations:
N67P, for example, in the RSV-A recombinant F protein Fm 1 as set forth in SEQ ID NO: 29;
L141P, for example, in the RSV-A recombinant F protein Fm 2 as set forth in SEQ ID NO: 30;
Q279P, for example, in the RSV-A recombinant F protein Fm 3 as set forth in SEQ ID NO: 31;
S377P, for example, in the RSV-A recombinant F protein Fm 4 as set forth in SEQ ID NO: 32;
N67P+L141P, for example, in the RSV-A recombinant F protein Fm 5 as set forth in SEQ ID NO: 33;
N67P+Q279P, for example, in the RSV-A recombinant F protein Fm 6 as set forth in SEQ ID NO: 34;
N67P+S377P, for example, in the RSV-A recombinant F protein Fm 7 as set forth in SEQ ID NO: 35;
L141P+Q279P, for example, in the RSV-A recombinant F protein Fm 8 as set forth in SEQ ID NO: 36;
L141P+S377P, for example, in the RSV-A recombinant F protein Fm 9 as set forth in SEQ ID NO: 37;
Q279P+S377P, for example, in the RSV-A recombinant F protein Fm 10 as set forth in SEQ ID NO: 38;
N67P+L141P+Q279P, for example, in the RSV-A recombinant F protein Fm 11 as set forth in SEQ ID NO: 39;
N67P+L141P+S377P, for example, in the RSV-A recombinant F protein Fm 12 as set forth in SEQ ID NO: 40;
N67P+Q279P+S377P, for example, in the RSV-A recombinant F protein Fm 13 as set forth in SEQ ID NO: 41;
L141P+Q279P+S377P, for example, in the RSV-A recombinant F protein Fm 14 as set forth in SEQ ID NO: 42;
N67P+L138P+G139P, for example, in the RSV-A recombinant F protein Fm 15 as set forth in SEQ ID NO: 43;
L138P+G139P+Q279P, for example, in the RSV-A recombinant F protein Fm 16 as set forth in SEQ ID NO: 44;
L138P+G139P+S377P, for example, in the RSV-A recombinant F protein Fm 17 as set forth in SEQ ID NO: 45;
N67P+L138P+G139P+Q279P, for example, in the RSV-A recombinant F protein Fm 18 as set forth in SEQ ID NO: 46;
N67P+L138P+G139P+S377P, for example, in the RSV-A recombinant F protein Fm 19 as set forth in SEQ ID NO: 47;
N67P+L141P+Q279P+S377P, for example, in the RSV-A recombinant F protein Fm 20 as set forth in SEQ ID NO: 48;
L138P+G139P+Q279P+S377P, for example, in the RSV-A recombinant F protein Fm 21 as set forth in SEQ ID NO: 49;
N67P+L138P+G139P+Q279P+S377P, for example, in the RSV-A recombinant F protein Fm 22 as set forth in SEQ ID NO: 50;
N67P+L138P+G139P+L141P+Q279P+S377P, for example, in the RSV-A recombinant F protein Fm 23 as set forth in SEQ ID NO: 51;
optionally, the N67P mutation in the above mutations or combinations of mutations may be replaced by the K65P mutation or D73P mutation;
optionally, the L141P mutation in the above mutations or combinations of mutations may be replaced by the L138P mutation or G139P mutation;
optionally, the above mutations or combinations of mutations further comprise one or more mutations selected from the group consisting of an S215P mutation, an E161P mutation, an I214P mutation, an N216P mutation, and a Q210P mutation.

For example, in some embodiments, the S215P mutation is further introduced into the above mutations, e.g., in the RSV-A recombinant F proteins Fm 26-34 with amino acid sequences as set forth in SEQ ID NOs: 54-62, respectively. In other embodiments, the E161P mutation is further introduced into the above mutations, for example, in the RSV-A recombinant F proteins Fm 35-46 with amino acid sequences as set forth in SEQ ID NOs: 63-74, respectively. In other embodiments, the I214P mutation is further introduced into the above mutations, for example, in the RSV-A recombinant F protein Fm 47 having an amino acid sequence as set forth in SEQ ID NO: 75. In other embodiments, the N216P mutation is further introduced into the above mutations, for example, in the RSV-A recombinant F protein Fm 48 having an amino acid sequence as set forth in SEQ ID NO: 76. In other embodiments, the Q210P mutation is further introduced into the above mutations, for example, in the RSV-A recombinant F proteins Fm 49-51 with amino acid sequences as set forth in SEQ ID NOs: 77-79, respectively.

In some embodiments, the RSV-A-F₁ peptide fragment and the RSV-A-F₂ peptide fragment are connected directly, for example, in the RSV-A recombinant F proteins Fm 70-72.

In other embodiments, the RSV-A-F₁ peptide fragment and the RSV-A-F₂ peptide fragment are connected by a linking bridge.

Preferably, the linking bridge is selected from the group consisting of:
(i) a (GS)m linking bridge, wherein m = 1-5, preferably 1-3, optionally comprising one or more amino acid mutations; for example, the linking bridge may be GS (as set forth in SEQ ID NO: 8, in the RSV-A recombinant F proteins Fm 65-67), or GSGSGRS (as set forth in SEQ ID NO: 7, in the RSV-A recombinant F proteins Fm 68-69);
(ii) a (GGGGS)n linking bridge, wherein n = 1-5, preferably 1-3, optionally comprising one or more mutations to proline. In particular embodiments, the linking bridge of this type is GGGGSGGGGSGGGGGS (i.e., SEQ ID NO: 2, e.g., in the RSV-A recombinant F proteins Fm 52-55 (as set forth in SEQ ID NOs: 80-83, respectively)), GGPGSGGGGSGGGGGS (i.e., SEQ ID NO: 3, for example, in the RSV-A recombinant F proteins Fm 1-51 (as set forth in SEQ ID NOs: 29-79, respectively)), GGGGSPGGGSGGGGGS (i.e., SEQ ID NO: 4, for example, in the RSV-A recombinant F proteins Fm 56 (as set forth in SEQ ID NO: 84)), GGGGPGGGGGSGGGGS (i.e., SEQ ID NO: 5, for example, in the RSV-A recombinant F proteins Fm 57 (as set forth in SEQ ID NO: 85)), or GGGGSGGGPSGGGGGS (SEQ ID NO: 6, e.g. in the RSV-A Recombinant F protein Fm 58 (as set forth in SEQ ID NO: 86));
(iii) the native linking sequence between the wild-type F₁ and F₂ peptide fragments, which is part of the F₀ protein itself, for example, a sequence as set forth in SEQ ID NO: 9 (e.g., in the RSV-A recombinant F proteins Fm 59-61 (as set forth in SEQ ID NOs: 87-89, respectively));
(iv) a sequence derived from the linking bridge (iii) by mutating the furin cleavage site thereon, for example, a sequence as set forth in SEQ ID NO: 10 (e.g., in the RSV-A recombinant F proteins Fm 62-64 (as set forth in SEQ ID NOs: 90-92, respectively)).

Preferably, the linking bridge comprises or consists of an amino acid sequence selected from the group consisting of SEQ ID NOs: 2-10.

In some preferred embodiments, the RSV-A recombinant F protein comprises or consists of an amino acid sequence selected from the group consisting of SEQ ID NOs: 29-100, or an amino acid sequence that is derived from any one of the amino acid sequences as set forth in SEQ ID NOs: 29-100 by the substitution, deletion, or addition of one or several amino acids therein and has the same or substantially the same immunogenicity as the amino acid sequence it is derived from.

Further, in some embodiments, the RSV-A recombinant F protein further comprises a trimerization tag.

Preferably, the trimerization tag is located at the C-terminus and has an amino acid sequence selected from the group consisting of SEQ ID NOs:24-27; for example, in the RSV-A recombinant F proteins Fm 73-76 (as set forth in SEQ ID NOs:101-104, respectively), the trimerization tags are as set forth in SEQ ID NO:24-27, respectively.

Optionally, a His-tag as set forth in SEQ ID NO: 28 may also be added at the C-terminus to facilitate subsequent protein isolation and purification.

In addition, in some embodiments, the RSV-A recombinant F protein further comprises a signal peptide.

Preferably, the signal peptide is located at the N-terminus and has an amino acid sequence selected from the group consisting of SEQ ID NOs: 11-23; for example, in the RSV-A recombinant F protein Fm 73, the signal peptide is as set forth in SEQ ID NO: 14; in the RSV-A recombinant F proteins Fm 74-76, the signal peptide is as set forth in SEQ ID NO: 11.

In a second aspect, the present application provides a polynucleotide which encodes the RSV-A recombinant F protein of the first aspect.

In specific embodiments, the polynucleotide is a human codon-optimized nucleotide sequence, which can be DNA or mRNA.

In some embodiments, the polynucleotide is a DNA molecule. Preferably, the DNA molecule comprises or consists of a DNA sequence as set forth in one of SEQ ID NOs: 105-180.

In other embodiments, the polynucleotide is an mRNA molecule. Preferably, the mRNA molecule comprises or consists of an RNA sequence corresponding to the DNA sequence as set forth in one of SEQ ID NOs: 105-180.

In a third aspect, the present application provides a nucleic acid construct which comprises the polynucleotide of the second aspect, and optionally at least one expression regulatory element operably linked to the polynucleotide.

In a fourth aspect, the present application provides an expression vector which comprises the nucleic acid construct of the third aspect.

In a fifth aspect, the present application provides a host cell into which the polynucleotide of the second aspect, the nucleic acid construct of the third aspect, or the expression vector of the fourth aspect is transformed or transfected.

Optionally, the host cell is a mammalian cell, an insect cell, a yeast cell, or a bacterial cell. Further optionally, the mammalian cell is a 293T cell, a 293F cell, or a CHO cell.

Further optionally, the bacterial cell is an *Escherichia coli* cell.

In a sixth aspect, the present application provides a respiratory syncytial virus subtype A (RSV-A) recombinant F protein trimer, which is formed by the polymerization of three RSV-A recombinant F proteins of the first aspect.

In a seventh aspect, the present application provides use of the RSV-A recombinant F protein of the first aspect, the polynucleotide of the second aspect, the nucleic acid construct of the third aspect, the expression vector of the fourth aspect, the host cell of the fifth aspect, or the RSV-A recombinant F protein trimer of the sixth aspect in the preparation of a vaccine for the prevention and/ treatment of respiratory syncytial virus infections.

In an eighth aspect, the present application provides a vaccine or immunogenic composition which comprises the RSV-A recombinant F protein of the first aspect, the polynucleotide of the second aspect, the nucleic acid construct of the third aspect, the expression vector of the fourth aspect, the host cell of the fifth aspect, or the RSV-A recombinant F protein trimer of the sixth aspect, and a physiologically acceptable vehicle, adjuvant, excipient, carrier, and/or diluent.

In some preferred embodiments, the vaccine or immunogenic composition is a respiratory syncytial virus recombinant protein vaccine which comprises the RSV-A recombinant F protein of the first aspect or the RSV-A recombinant F protein trimer of the sixth aspect, and an adjuvant.

Optionally, the adjuvant is one or more selected from the group consisting of an aluminum adjuvant, an MF59 adjuvant, an MF59-like adjuvant, and AS series-like adjuvants.

In other preferred embodiments, the vaccine or immunogenic composition is a respiratory viral syncytial virus DNA vaccine which comprises:
(1) a eukaryotic expression vector; and
(2) a DNA sequence encoding the RSV-A recombinant F protein of the first aspect, preferably a DNA sequence as set forth in one of SEQ ID NOs: 105-180, which is constructed into the eukaryotic expression vector.

Optionally, the eukaryotic expression vector is selected from the group consisting of pGX0001, pVAX1, pCAGGS, and pcDNA series vectors.

In other preferred embodiments, the vaccine or immunogenic composition is a respiratory viral syncytial virus mRNA vaccine which comprises:
(I) an mRNA sequence encoding the RSV-A recombinant F protein of the first aspect, preferably an mRNA sequence corresponding to the DNA sequence as set forth in one of SEQ ID NOs: 105-180; and
(II) a lipid nanoparticle.

In another preferred embodiment, the vaccine or immunogenic composition is a respiratory viral syncytial virus-viral vector vaccine which comprises:
(1) a viral backbone vector; and
(2) a DNA sequence encoding the RSV-A recombinant F protein of the first aspect, preferably a DNA sequence as set forth in one of SEQ ID NOs: 105-180, which is constructed into the viral backbone vector.

Optionally, the viral backbone vector is one or more selected from the following viral vectors: an adenovirus vector, a poxvirus vector, an influenza virus vector, and an adeno-associated virus vector.

In one possible embodiment, the vaccine or immunogenic composition is in the form of a nasal spray, an oral formulation, a suppository, or a parenteral formulation.

Preferably, the nasal spray is selected from the group consisting of an aerosol, a spray, and a powder inhalant.

Preferably, the oral formulation is selected from the group consisting of a tablet, a powder, a pill, a pulvis, a granule, a fine granule, a soft/hard capsule, a film-coated preparation, a pellet, a sublingual tablet, and an ointment.

Preferably, the parenteral formulation is a transdermal formulation, an ointment, a plaster, a topical liquid, or an injectable or bolus-injectable formulation.

In a ninth aspect, the present application provides a method for preparing the RSV-A recombinant F protein of the first aspect, which comprises:
adding a nucleotide sequence encoding a signal peptide to the 5' end of the codon-optimized nucleotide sequence encoding the RSV-A recombinant F protein of the first aspect and adding nucleotide sequences encoding a trimerization tag and a His-tag as well as a stop codon to the 3' end thereof, performing cloning and expression, screening for correct recombinants, and then transfecting the correct recombinants into an expression system cell for protein expression, collecting the cell culture supernatant, and isolating the RSV-A recombinant F protein therefrom.

In one possible embodiment of the above method, the expression system cell is a mammalian cell, an insect cell, a yeast cell, or a bacterial cell; optionally, the mammalian cell is a 293T cell, a 293F cell, or a CHO cell; optionally, the bacterial cell is an *Escherichia coli* cell.

### Beneficial effects

When the natural RSV-A virus F protein antigen is recombinantly expressed *in vitro,* it is impossible to obtain a F antigen protein with pre-fusion conformation since the pre-fusion conformation is unstable. In the present application, by introducing one or more mutations to proline into the F₁ and/or F₂ peptide fragments of a respiratory syncytial virus A subtype F protein, a stable, pre-fusion conformation of RSV-A recombinant F protein is obtained. Experimental results show that the resulting RSV-A recombinant F protein comprises at least one epitope specific to the pre-fusion F protein, and can be expressed stably in a uniform form and with a significantly increased yield. In addition, the RSV-A recombinant F protein of the present application has strong immunogenicity and thus can stimulate the body to produce a high-level antibody titer (after booster immunization with the recombinant protein without adjuvant, the induced antigen-specific antibody titer can reach more than 10,000, and can be further increased by 10- to 100-fold when AddaVax adjuvant is incorporated), which is of great significance for the clinical treatment, prevention, and control of respiratory syncytial virus infections.

### Brief Description of the Drawings

One or more examples are exemplarily illustrated by reference to the accompanying drawings and these exemplary illustrations do not limit the embodiments. As used herein, the term "exemplary" means "serving as an example, instance, or illustration". Any embodiment described herein as "exemplary" should not be construed as superior or better than other embodiments.

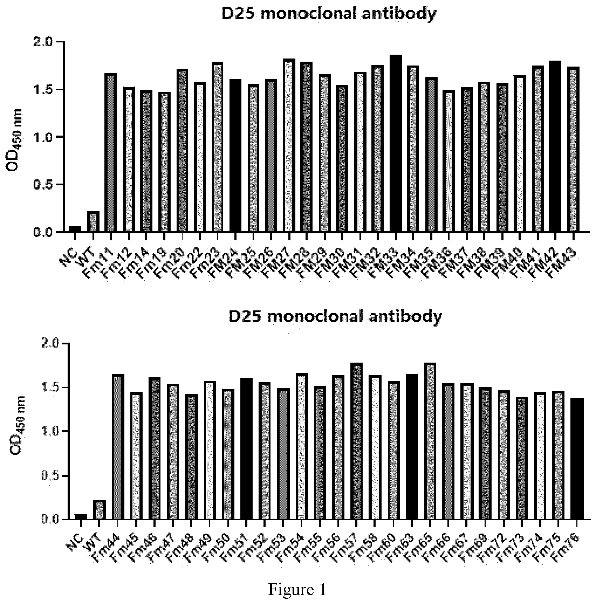

Figure 1 shows the expression of RSV-A recombinant F proteins in the supernatant stock solution detected by ELISA using a D25 monoclonal antibody, as recited in Example 3, wherein the abscissa indicates the experimental groups (i.e., the tested RSV-A recombinant F proteins), and the ordinate indicates the absorbance values at OD₄₅₀ nm.

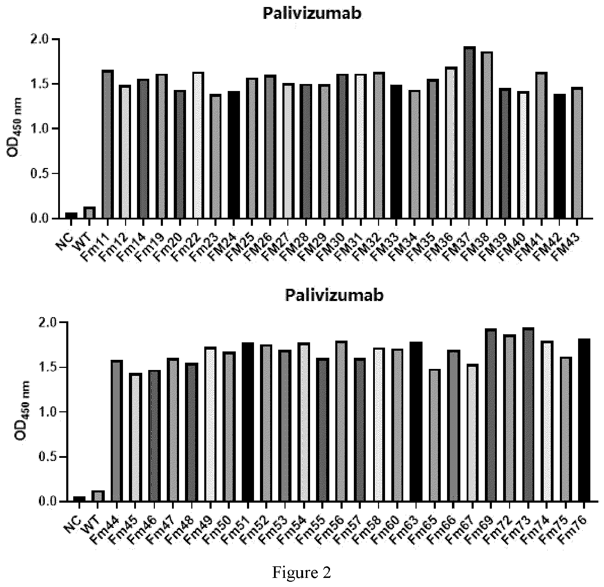

Figure 2 shows the expression of RSV-A recombinant F proteins in the supernatant stock solution detected by ELISA using palivizumab, as recited in Example 3, wherein the abscissa indicates the experimental groups (i.e., the tested RSV-A recombinant F proteins), and the ordinate indicates the absorbance values at OD₄₅₀ nm.

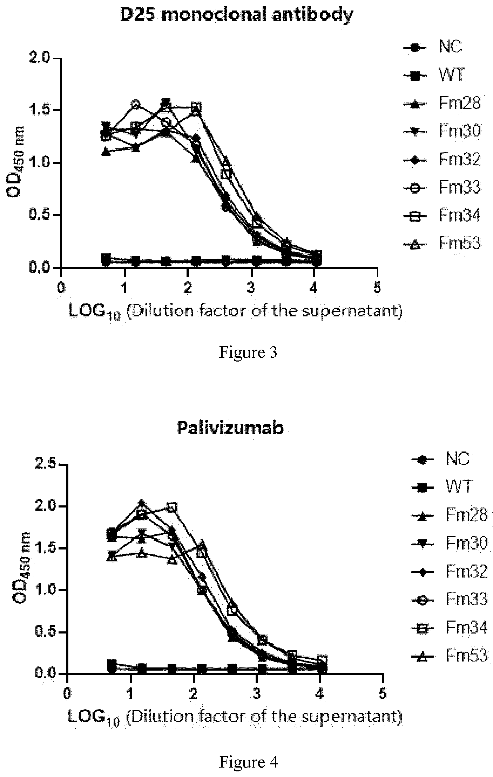

Figure 3 shows the expression of partial of the RSV-A recombinant F proteins in the diluted supernatants detected by ELISA using a D25 monoclonal antibody, as recited in Example 3, wherein the abscissa indicates the Log10 values of the dilution factors of the cell supernatants, the ordinate indicates the absorbance values at OD₄₅₀ nm, and the right legend shows the experimental groups (i.e., the tested RSV-A recombinant F proteins).
Figure 4 shows the expression of part of the RSV-A recombinant F proteins in the diluted supernatants detected by ELISA using palivizumab, as recited in Example 3, wherein the abscissa indicates the Log10 values of the dilution factors of the cell supernatants, the ordinate indicates the absorbance values at OD₄₅₀ nm, and the right legend shows the experimental groups (i.e., the tested RSV-A recombinant F proteins).

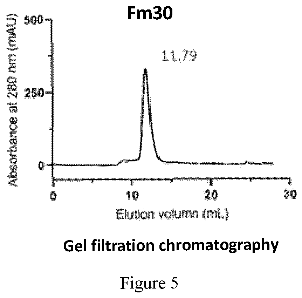

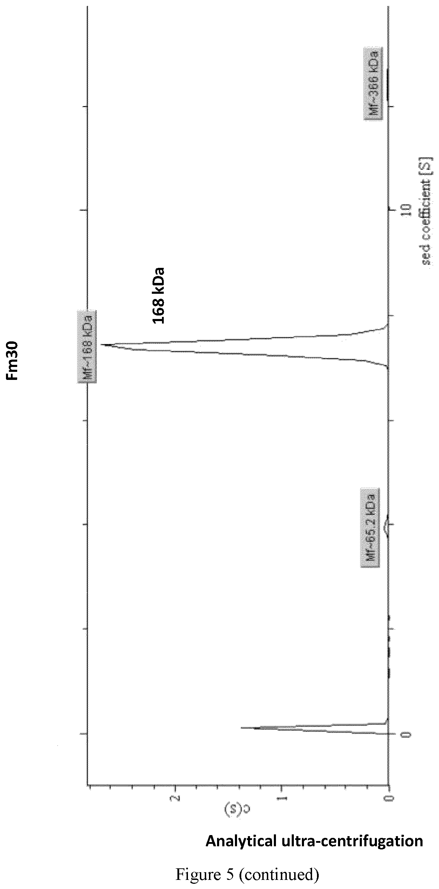

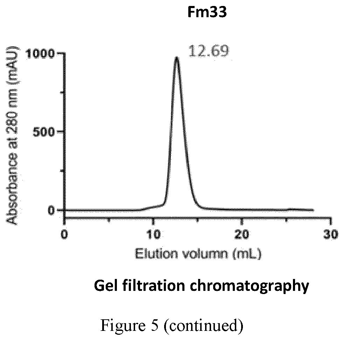

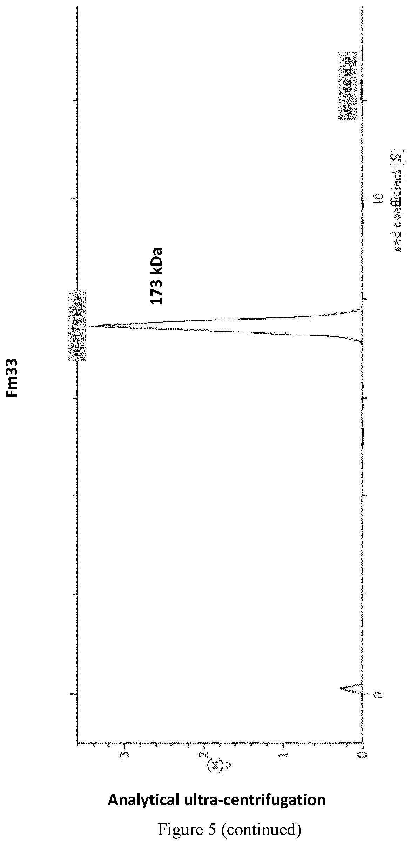

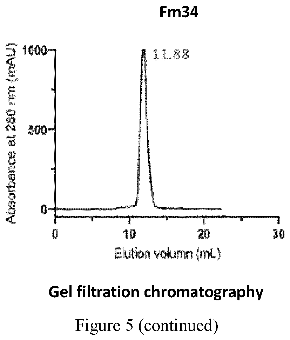

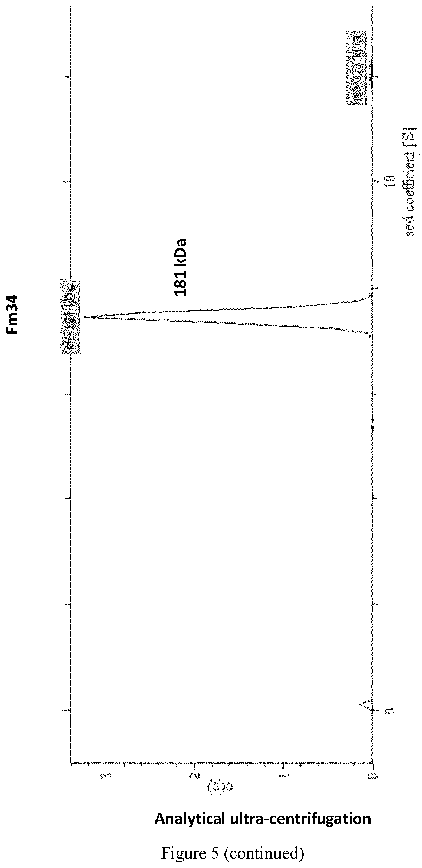

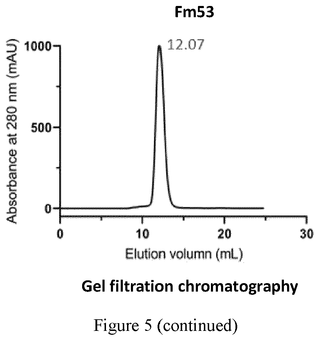

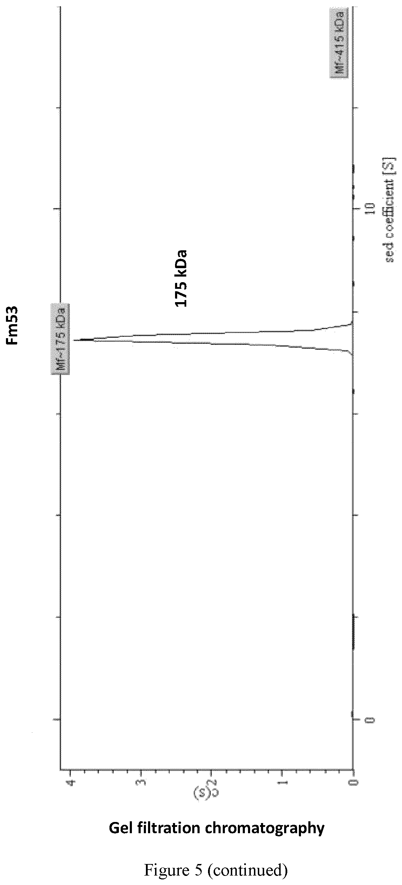

Figure 5 shows the results of the purification of the RSV-A recombinant F proteins from the expression supernatant by molecular sieve chromatography, and the results of analytical ultracentrifugation of the purified proteins, as recited in Example 4, wherein the left panels display the UV absorption profiles during the purification of the RSV-A recombinant F proteins from the expression supernatant by molecular sieve chromatography, and the right panels show the results of analytical ultracentrifugation of the purified RSV-A recombinant F proteins.

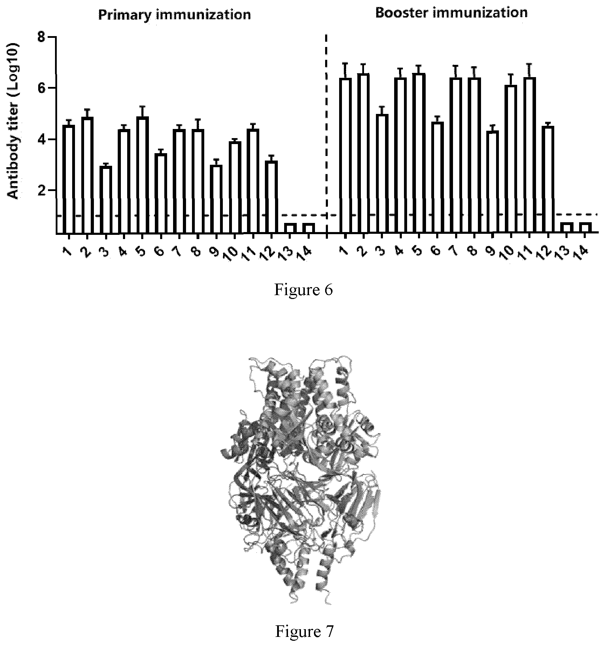

Figure 6 shows, as recited in Example 6 and detected by ELISA, the titer levels of specific IgG antibodies in the sera of mice following primary and booster immunizations with the RSV-A recombinant F proteins as immunogens as described in Example 5, wherein the abscissa indicates the groups listed in Table 1, and the ordinate indicates the antibody titers.
Figure 7 shows a structural diagram of the RSV-A recombinant F protein Fm 30, as resolved in Example 7.

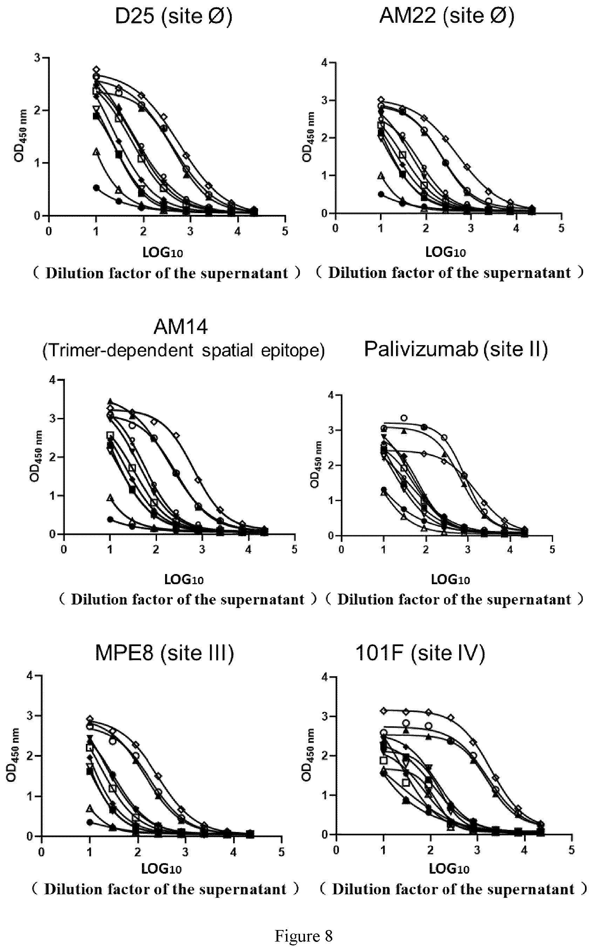

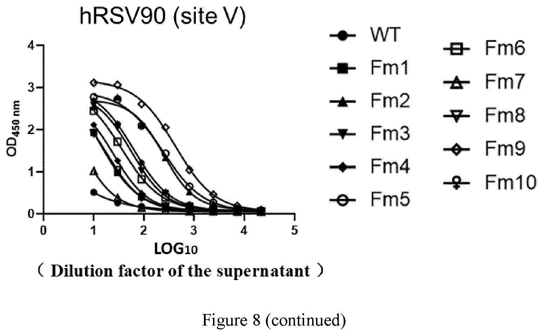

Figure 8 shows the expression of the recombinant F proteins RSV-A-Fm 1-10 and the wild-type RSV-A F protein (WT) in the diluted supernatants detected by ELISA using monoclonal antibodies D25, AM22, AM14, palivizumab, MPE8, 101F, and hRSV90, and the binding of the respective recombinant F proteins to each of the above antibodies, as recited in Example 9, wherein the abscissa indicates the Log10 values of the dilution factors of the cell supernatants, the ordinate indicates the absorbance values at OD₄₅₀ nm, and the right legend shows the experimental groups (i.e., the tested RSV-A recombinant F proteins).

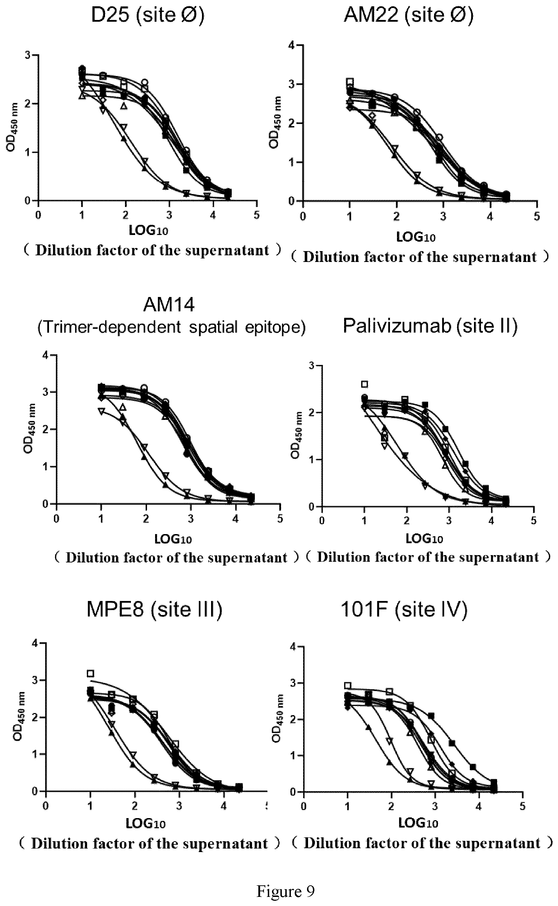

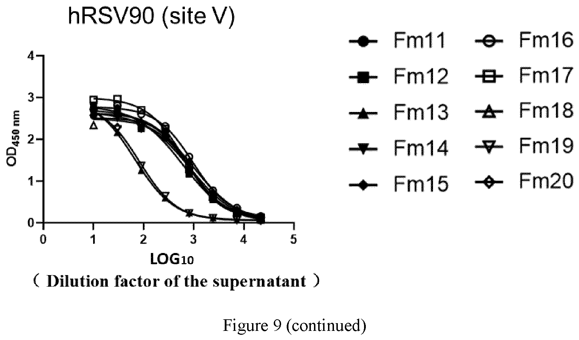

Figure 9 shows the expression of the recombinant F proteins RSV-A-Fm 11-20 in the diluted supernatants detected by ELISA using monoclonal antibodies D25, AM22, AM14, palivizumab, MPE8, 101F, and hRSV90, and the binding of the respective recombinant F proteins to each of the above antibodies, as recited in Example 9, wherein the abscissa indicates the Log10 values of the dilution factors of the cell supernatants, the ordinate indicates the absorbance values at OD₄₅₀ nm, and the right legend shows the experimental groups (i.e., the tested RSV-A recombinant F proteins).

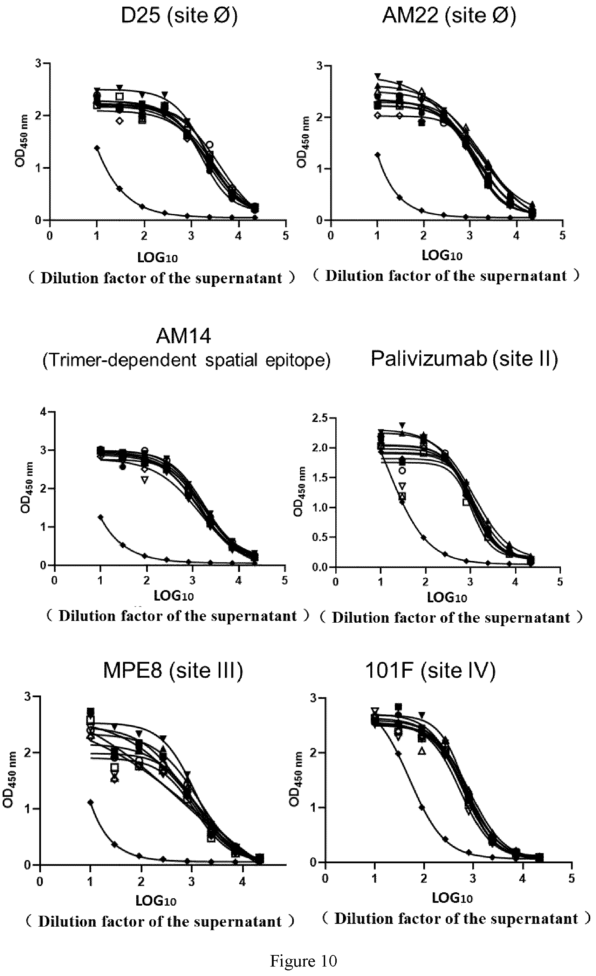

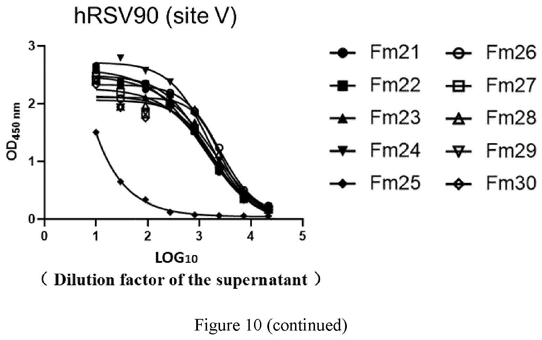

Figure 10 shows the expression of the recombinant F proteins RSV-A-Fm 21-30 in the diluted supernatants detected by ELISA using monoclonal antibodies D25, AM22, AM14, palivizumab, MPE8, 101F, and hRSV90, and the binding of the respective recombinant F proteins to each of the above antibodies, as recited in Example 9, wherein the abscissa indicates the Log10 values of the dilution factors of the cell supernatants, the ordinate indicates the absorbance values at OD₄₅₀ nm, and the right legend shows the experimental groups (i.e., the tested RSV-A recombinant F proteins).

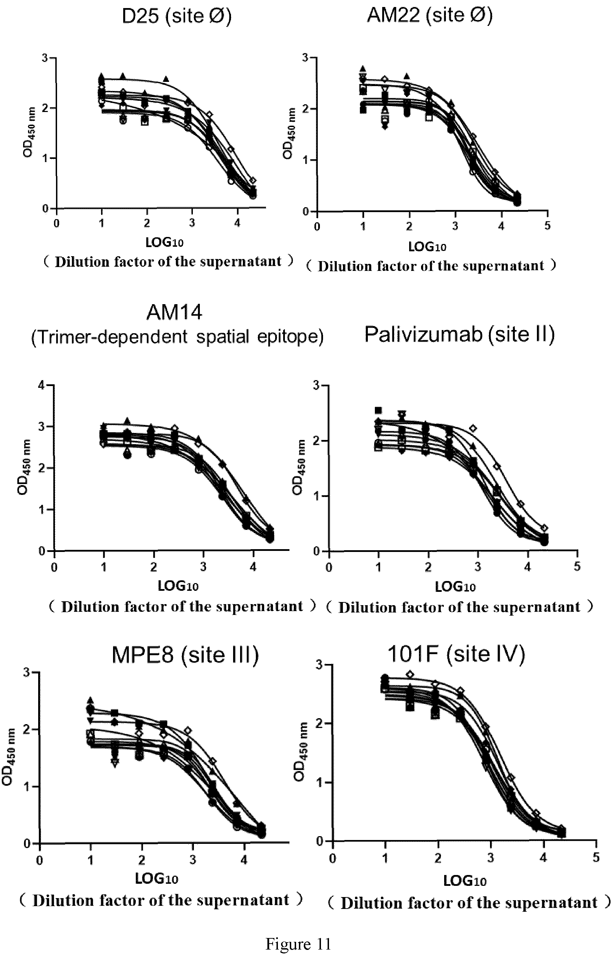

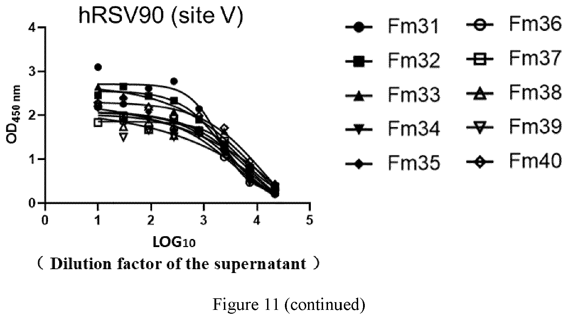

Figure 11 shows the expression of the recombinant F proteins RSV-A-Fm 31-40 in the diluted supernatant detected by ELISA using monoclonal antibodies D25, AM22, AM14, palivizumab, MPE8, 101F, and hRSV90, and the binding of the respective recombinant F proteins to each of the above antibodies, as recited in Example 9, wherein the abscissa indicates the Log10 values of the dilution factors of the cell supernatants, the ordinate indicates the absorbance values at OD₄₅₀ nm, and the right legend shows the experimental groups (i.e., the tested RSV-A recombinant F proteins).

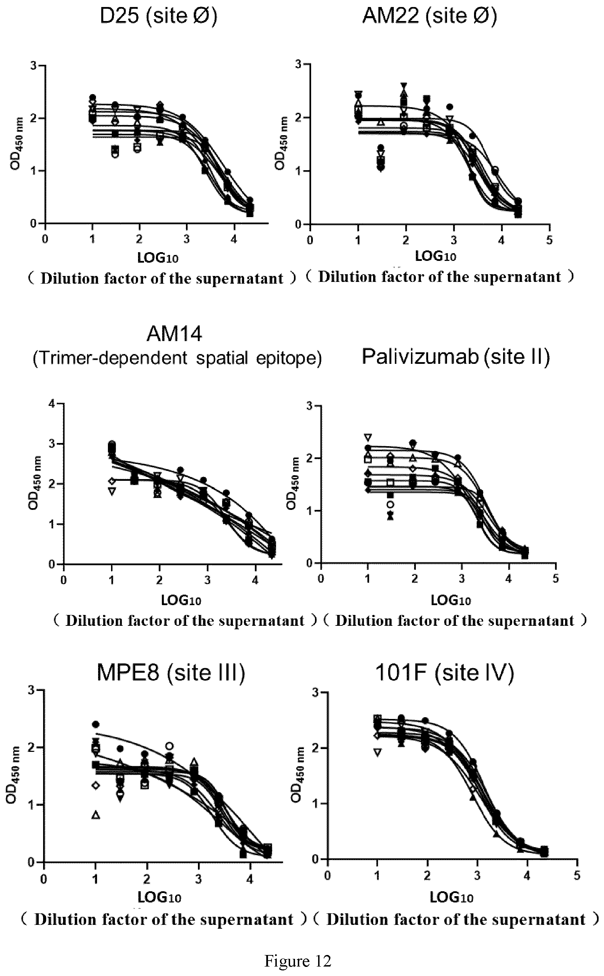

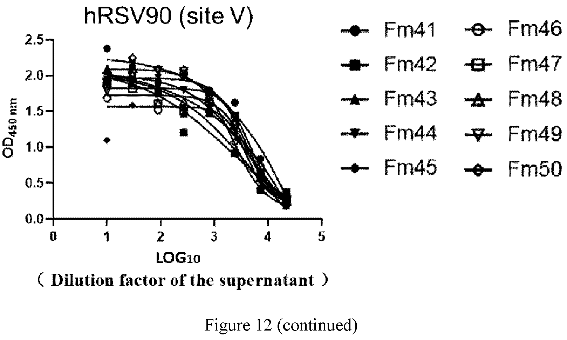

Figure 12 shows the expression of the recombinant F proteins RSV-A-Fm 41-50 in the diluted supernatants detected by ELISA using monoclonal antibodies D25, AM22, AM14, palivizumab, MPE8, 101F, and hRSV90, and the binding of the respective recombinant F proteins to each of the above antibodies, as recited in Example 9, wherein the abscissa indicates the Log10 values of the dilution factors of the cell supernatants, the ordinate indicates the absorbance values at OD₄₅₀ nm, and the right legend shows the experimental groups (i.e., the tested RSV-A recombinant F proteins).

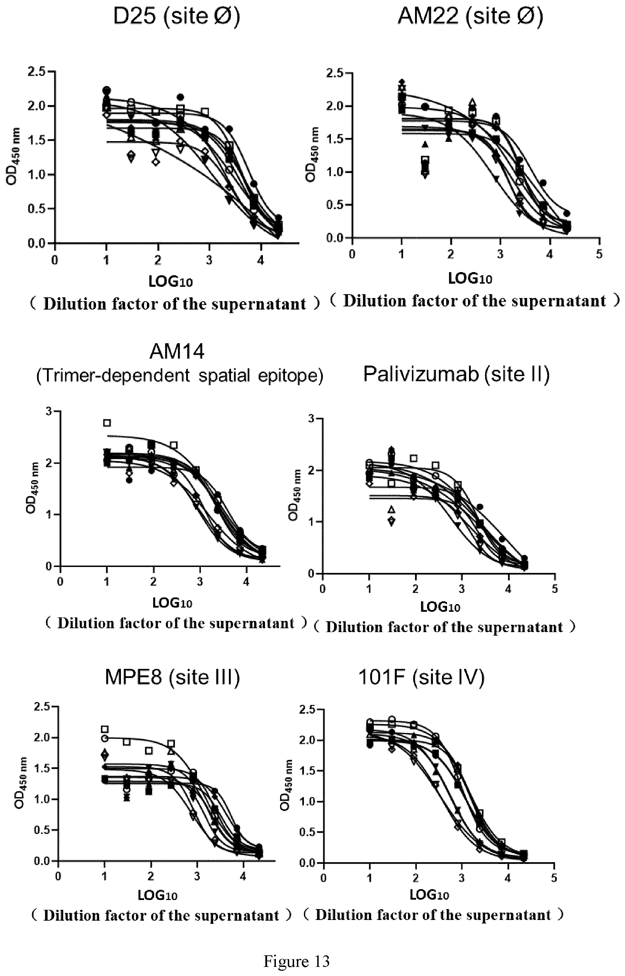

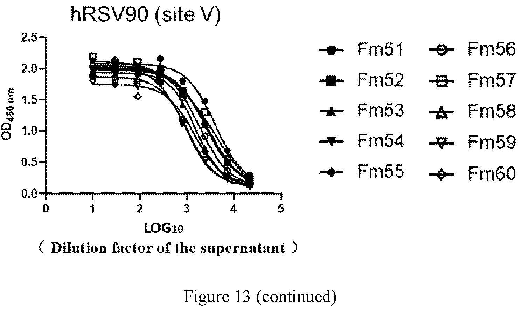

Figure 13 shows the expression of the recombinant F proteins RSV-A-Fm 51-60 in the diluted supernatant detected by ELISA using monoclonal antibodies D25, AM22, AM14, palivizumab, MPE8, 101F, and hRSV90, and the binding of the respective recombinant F proteins to each of the above antibodies, as recited in Example 9, wherein the abscissa indicates the Log10 values of the dilution factors of the cell supernatants, the ordinate indicates the absorbance values at OD₄₅₀ nm, and the right legend shows the experimental groups (i.e., the tested RSV-A recombinant F proteins).

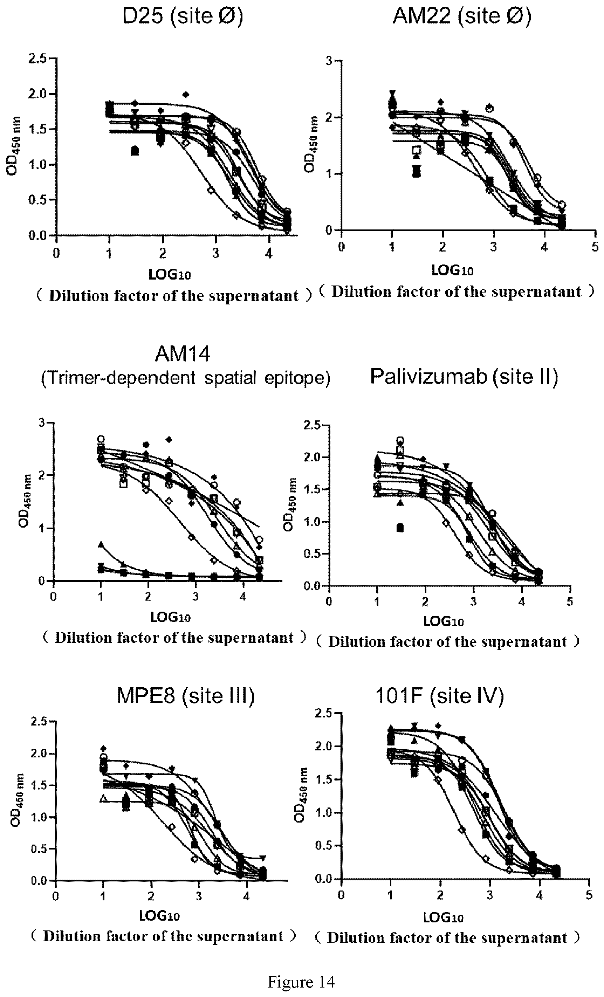

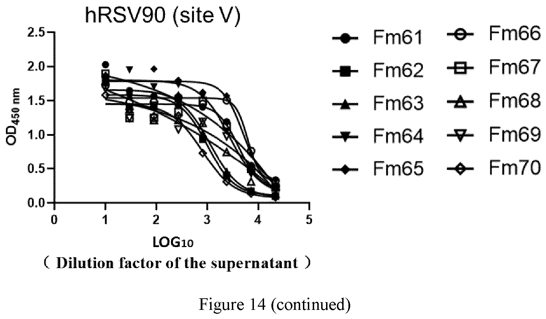

Figure 14 shows the expression of the recombinant F proteins RSV-A-Fm 61-70 in the diluted supernatant detected by ELISA using monoclonal antibodies D25, AM22, AM14, palivizumab, MPE8, 101F, and hRSV90, and the binding of the respective recombinant F proteins to each of the above antibodies, as recited in Example 9, wherein the abscissa indicates the Log10 values of the dilution factors of the cell supernatants, the ordinate indicates the absorbance values at OD₄₅₀ nm, and the right legend shows the experimental groups (i.e., the tested RSV-A recombinant F proteins).

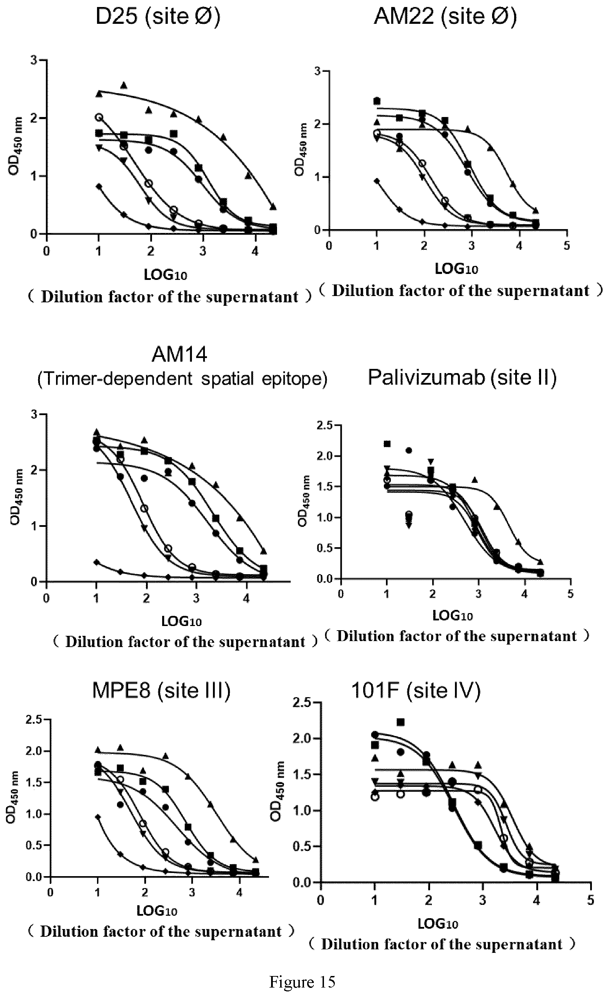

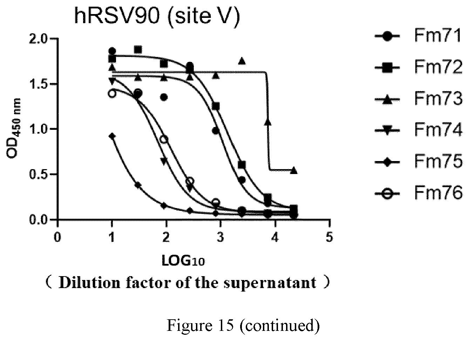

Figure 15 shows the expression of the recombinant F proteins RSV-A-Fm 71-76 in the diluted supernatant detected by ELISA using monoclonal antibodies D25, AM22, AM14, palivizumab, MPE8, 101F, and hRSV90, and the binding of the respective recombinant F proteins to each of the above antibodies, as recited in Example 9, wherein the abscissa indicates the Log10 values of the dilution factors of the cell supernatants, the ordinate indicates the absorbance values at OD₄₅₀ nm, and the right legend shows the experimental groups (i.e., the tested RSV-A recombinant F proteins).

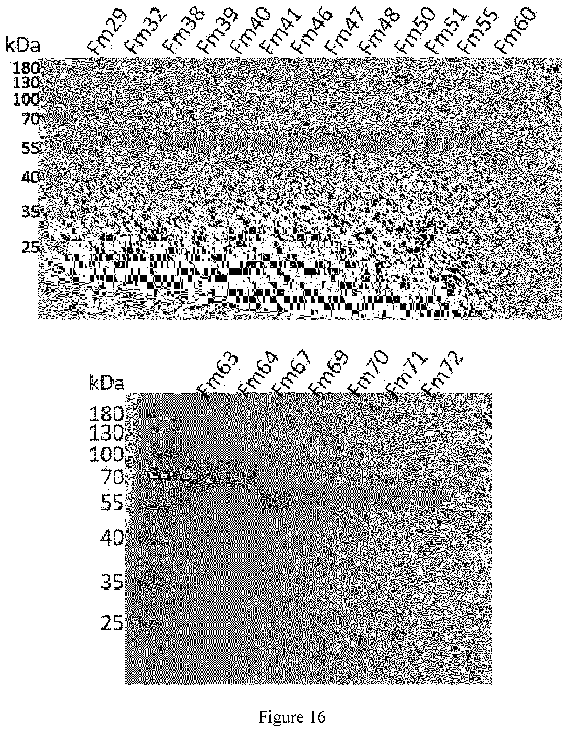

Figure 16 shows SDS-PAGE detection results of the isolated and purified recombinant F proteins RSV-A-Fm 29, 32, 38, 39, 40, 41, 46, 47, 48, 50, 51, 55, 60, 63, 64, 67, 69, 70, 71, and 72 , as recited in Example 10.

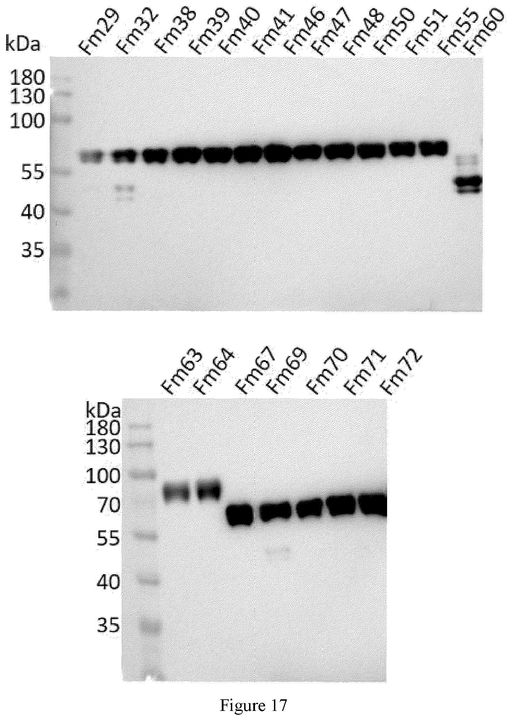

Figure 17 shows Western Blot detection results of the isolated and purified recombinant F proteins RSV-A-Fm 29, 32, 38, 39, 40, 41, 46, 47, 48, 50, 51, 55, 60, 63, 64, 67, 69, 70, 71, and 72 , as recited in Example 10.

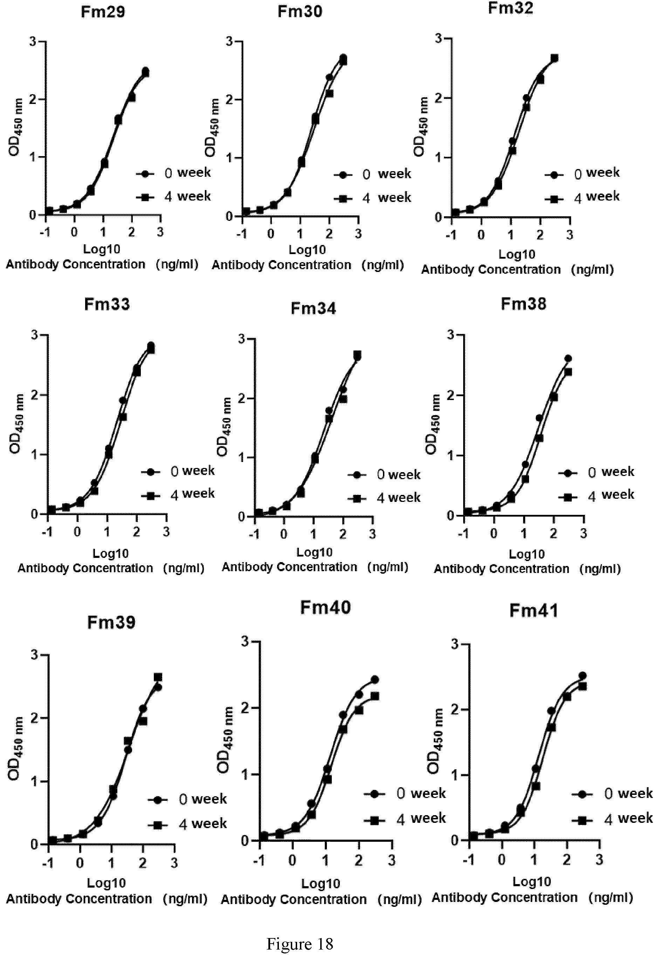

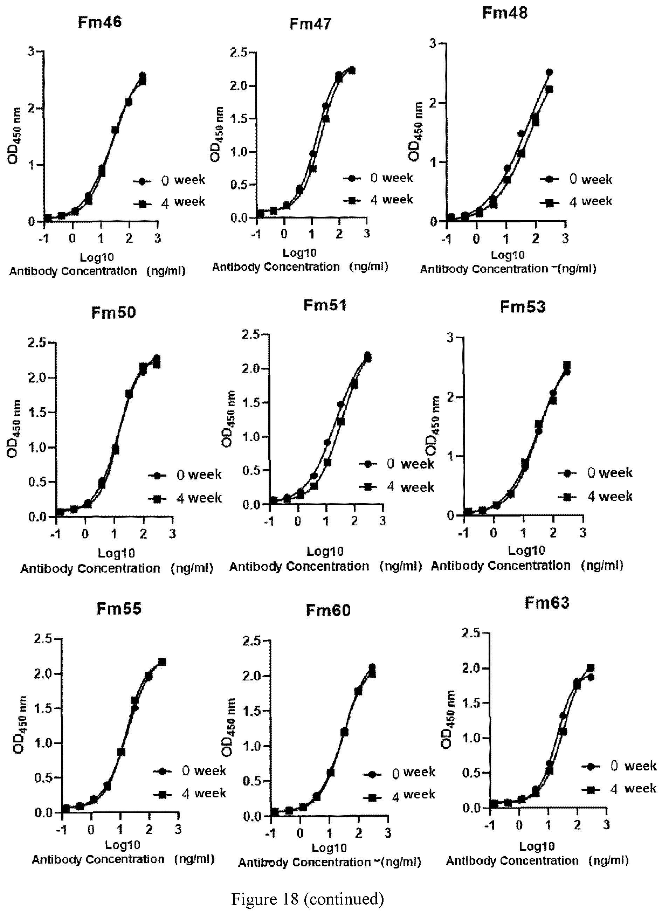

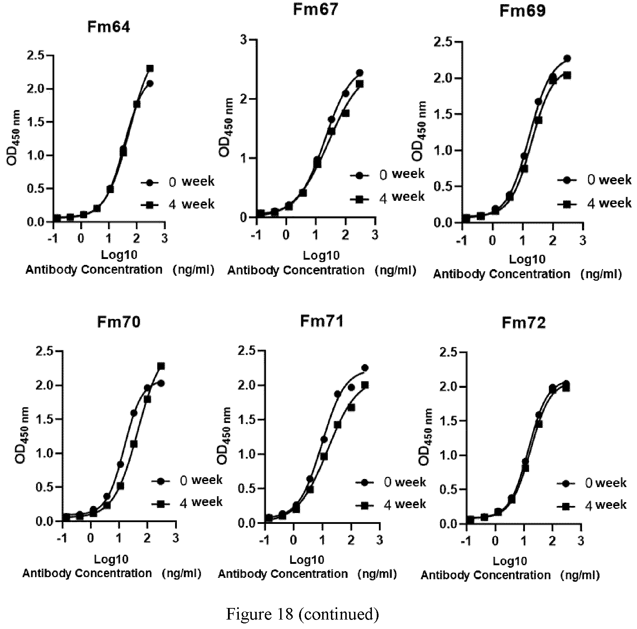

Figure 18 shows the binding of the recombinant F proteins RSV-A-Fm 29, 30, 32, 33, 34, 38, 39, 40, 41, 46, 47, 48, 50, 51, 53, 55, 60, 63, 64, 67, 69, 70, 71, and 72 after storage at 30 °C for 4 weeks to the monoclonal antibody AM22 detected by ELISA, as recited in Example 11, wherein the abscissa indicates the protein concentrations, and the ordinate indicates the absorbance values at OD₄₅₀ nm.

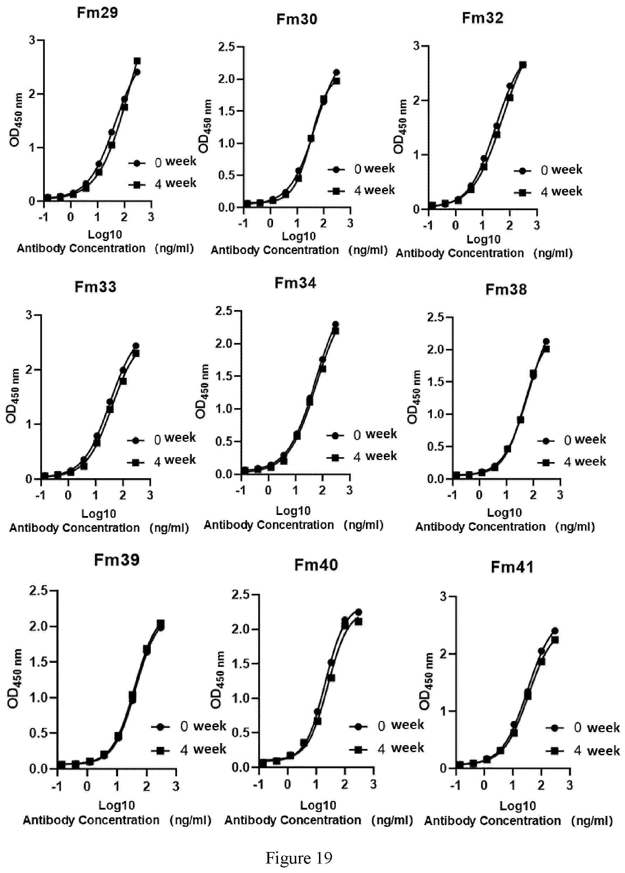

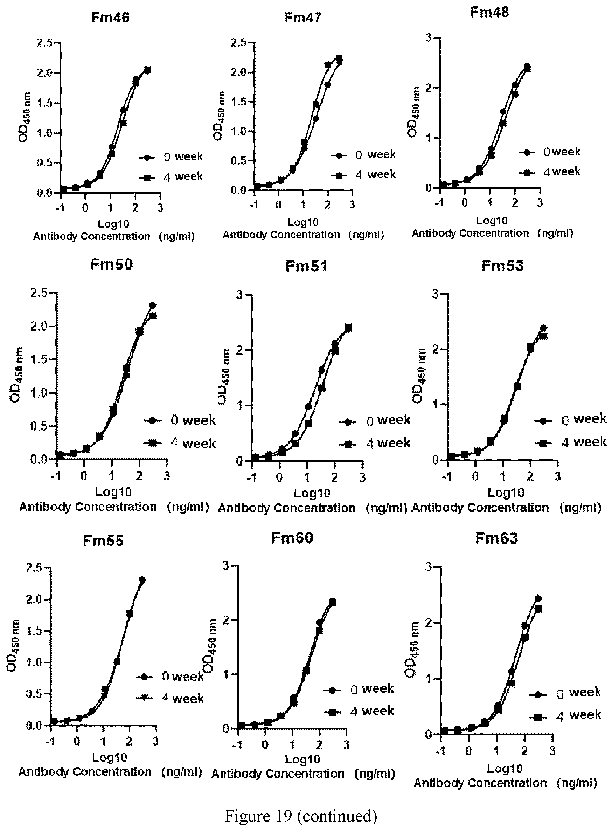

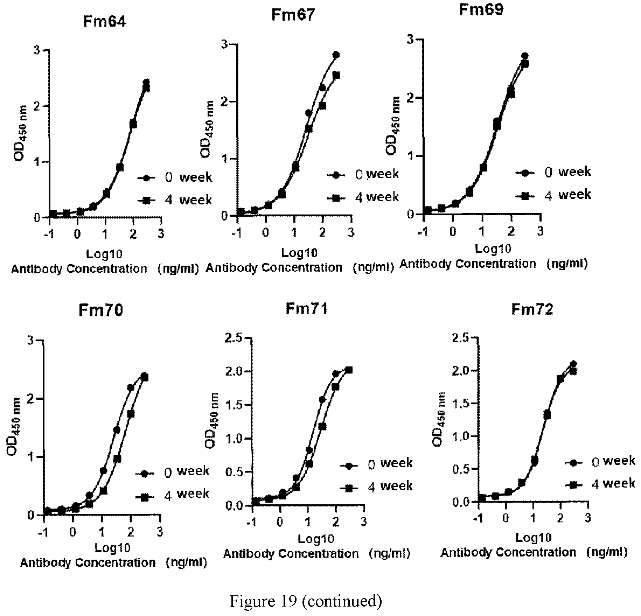

Figure 19 shows the binding of the recombinant F proteins RSV-A-Fm 29, 30, 32, 33, 34, 38, 39, 40, 41, 46, 47, 48, 50, 51, 53, 55, 60, 63, 64, 67, 69, 70, 71, and 72 after storage at 30 °C for 4 weeks to palivizumab detected by ELISA, as recited in Example 11, wherein the abscissa indicates the protein concentrations, and the ordinate indicates the absorbance values at OD450 nm.

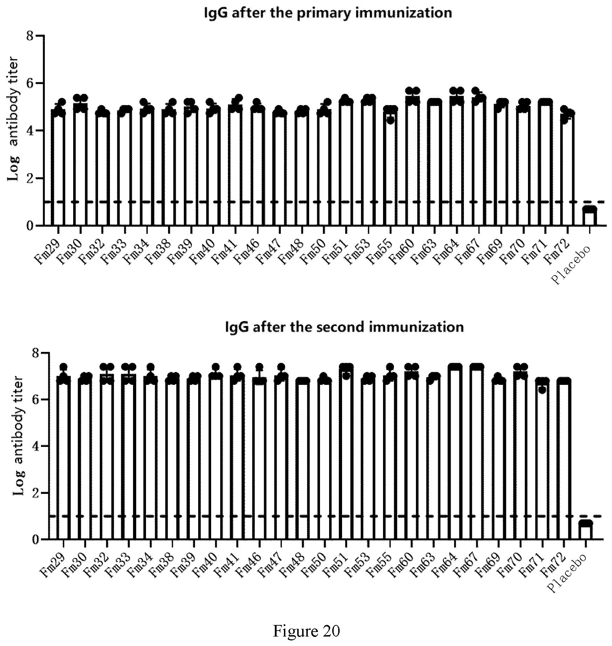

Figure 20 shows, as recited in Example 13 and detected by ELISA, the titer levels of specific binding IgG antibodies in the sera of mice following primary immunization and booster immunizations with the recombinant F proteins RSV-A FM 29, 30, 32, 33, 34, 38, 39, 40, 41, 46, 47, 48, 50, 51, 53, 55, 60, 63, 64, 67, 69, 70, 71, and 72 as immunogens, respectively, as described in Example 12, wherein the abscissa indicates the vaccine groups, and the ordinate indicates the antibody titers.

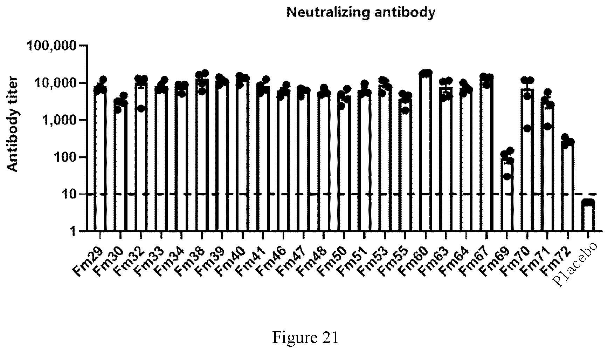

Figure 21 shows, as recited in Example 14 and detected by trace neutralization assay, the levels of neutralizing antibodies against respiratory syncytial virus in the sera of mice following booster immunization with the recombinant F proteins RSV-A-FM 29, 30, 32, 33, 34, 38, 39, 40, 41, 46, 47, 48, 50, 51, 53, 55, 60, 63, 64, 67, 69, 70, 71, and 72 as immunogens, respectively, as described in Example 12, wherein the abscissa represents the vaccine groups, and the ordinate represents the neutralizing antibody titers.

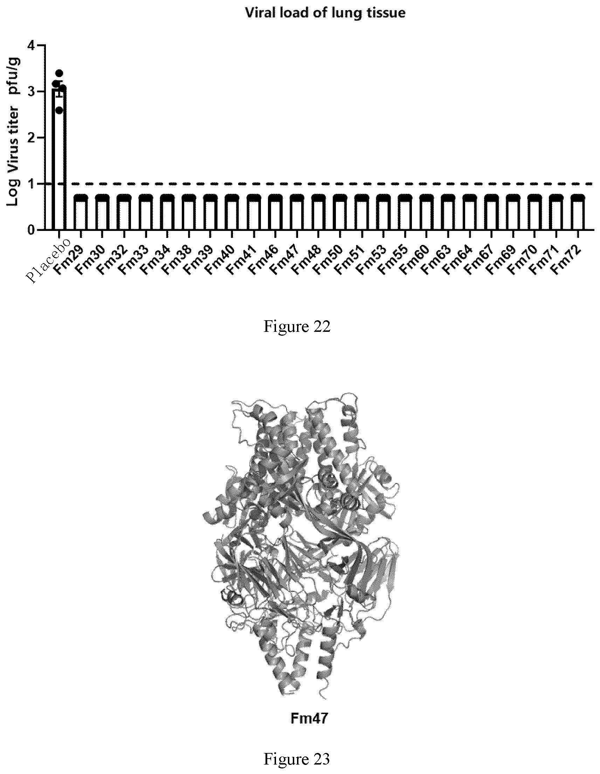

Figure 22 shows, as recited in Example 15 and detected by plaque assay, the viral loads of lung tissues of mice immunized via nasal drop with the respective vaccines after challenge with respiratory syncytial virus as described in Example 12, wherein the abscissa represents the vaccine groups and the ordinate represents the viral loads.
Figure 23 shows the structural diagram of the recombinant F protein RSV-A Fm 47, as resolved in Example 16.

### Detailed Description of the Invention

To clarify the purpose, technical solutions, and advantages of the invention, the embodiments of the invention will be described clearly and comprehensively in the following sections. It should be noted that the described embodiments represent only a portion of the embodiments of the present invention and not all possible implementations. Based on the embodiments disclosed herein, any other embodiments that can be derived by those of ordinary skill in the art without inventive effort shall fall within the scope of the present invention.

Furthermore, to facilitate a better understanding of the present invention, numerous specific details are provided in the following embodiments. However, it will be understood by those skilled in the art that the invention may be practiced without certain specific details. In some instances, well-known materials, components, methods, or techniques have not been described in detail to avoid obscuring the essence of the invention.

Throughout the specification and claims, the term "comprising" or variations thereof, such as "including" or "containing," shall be interpreted as encompassing the stated elements or steps without excluding the presence of additional elements or steps, unless explicitly stated otherwise.

### Example 1: Construction of expression plasmids for RSV-A recombinant F proteins of the present application

In this example, 76 kinds of RSV-A recombinant F proteins of the present application were designed and constructed, designated as RSV-A-Fm 1 through RSV-A-Fm 76, with their amino acid sequences set forth in SEQ ID NOs: 29-104, respectively. For the RSV-A-Fm 1-72 (as shown in SEQ ID NOs: 29-100, respectively), a signal peptide sequence of SEQ ID NO: 11 was added to the N-terminus thereof, and a trimerization tag of SEQ ID NO: 24 and a His-tag of SEQ ID NO: 28 were added to the C- terminus thereof. In contrast, the RSV-A-Fm 73-76 (as shown in SEQ ID NOs: 101-104) inherently included a signal peptide at the N-terminus and a trimerization tag sequence followed by a His-tag sequence at the C-terminus.

According to the codon preference of mammalian cells, the nucleic acid sequences encoding the above RSV-A recombinant F proteins (RSV-A-FM 1-76) were optimized to obtain the optimized coding sequences which were as set forth in SEQ ID NOs: 105-180, respectively. For the sequences as set forth in SEQ ID NOs: 105-176, the EcoRI restriction site, the Kozak sequence, and the nucleic acid sequence encoding the signal peptide were added to the 5' end thereof, and the nucleic acid sequences encoding the trimerization tag, the His-tag, a stop codon, and the XhoI restriction site were added to the 3' end thereof. The resultant sequences were synthesized by Nanjing Kingsley Biotechnology Co., Ltd, and subsequently cloned into a pCAGGS vector through the EcoRI and XhoI restriction sites, resulting in the expression plasmids for the recombinant proteins RSV-A-Fm 1-72. For the sequences as set forth in SEQ ID NOs: 177-180, the EcoRI restriction site and the Kozak sequence were added to the 5' end thereof, and a stop codon and the XhoI restriction site were added to the 3' end thereof. The resultant sequences were synthesized by Nanjing Kingsley Biotechnology Co., Ltd, and subsequently cloned into a pCAGGS vector through the EcoRI and XhoI restriction sites, resulting in the expression plasmids for the recombinant proteins RSV-A-Fm 73-76.

### Example 2: Expression, isolation, and purification of detection antibodies

In this example, according to the methods for constructing antibody heavy chain and light chain sequences and their expression plasmids disclosed in the literatures (Z. Wei et al., Analytical Chemistry 79, 2797-2805 (2007); Q. Zhu et al., Science Translational Medicine 9, (2017)), expression plasmids for the heavy and light chains of the detection antibodies against RSV F protein, namely the D25 monoclonal antibody and palivizumab, were constructed respectively.

### Antibody expression

14-16 hours before transfection, 293T cells at a relatively high density were subcultured, for example, 293T cells in a 10-cm culture dish with 100% confluence were passaged at a ratio of 1:3. 14-16 hours later, when the cell density reached more than 70%, transfection was carried out. For transfection, the heavy chain and light chain plasmids of the antibody were co-transfected into 293T cells at a ratio of 2:3. After 4-6 hours of transfection, the cells were washed twice with PBS, and then cultured in serum-free DMEM medium. The cell supernatants were collected on day 3 and day 7 post-transfection, respectively, and subjected to centrifugation to remove cell debris. The antibody-containing supernatants from both collections were pooled for subsequent purification of antibody proteins.

### Antibody purification

A Protein A (5 ml) HP affinity column (GE Healthcare) was connected to an AKTA Purifier/Explorer/FPLC/START system (GE Healthcare), and the purification process was performed on the instrument as follows: 20% ethanol in the column was flushed out with water, and then the column was equilibrated with 20 mM Na₃PO₄ buffer at pH 7.0 until the conductivity of the instrument became stable, at which the above antibody-containing supernatant was loaded onto the column via a 10 mL loop at a flow rate of 2 mL/min to allow binding to Protein A. After the UV became stable, approximately 0.8 mL of 1 M Tris buffer at pH 9.0 was added to the collection tube (with a collection volume of approximately 3.2 mL), and then the procedure was switched to using 100% 0.1 M Gly buffer at pH 3.0 to elute the antibodies bound to the column. The eluate was collected, then the antibody buffer was exchanged to PBS by a concentration and buffer exchange method. The obtained antibody solution could be used directly or aliquoted and stored in a -80 °C freezer for later use.

### Example 3: Expression and conformational characterization of RSV-A-Fm recombinant proteins

In this example, HEK293T cells were transfected with expression plasmids for partial RSV-A-Fm recombinant proteins (Fm 11, 12, 14, 19, 20, 22 - 58, 60, 63, 65 - 67, 69, 72 - 76) as constructed in Example 1 to express the corresponding RSV-A-Fm recombinant proteins. Then, the expressed recombinant proteins were detected by ELISA using the detection antibodies D25 monoclonal antibody and palivizumab as prepared in Example 2, respectively, to determine the conformation thereof.

Specifically, HEK293T cells were cultured in DMEM medium supplemented with 10% FBS. When the cell density reached more than 70%, transfection was performed. The HEK293T cells were transfected with the expression plasmids for the RSV-A-Fm recombinant proteins Fm 11, 12, 14, 19, 20, 22-58, 60, 63, 65-67, 69, 72-76 as constructed in Example 1. After 4-6 hours of transfection, the cell culture medium was replaced with serum-free DMEM and the cells were cultured for another 3 days. Then the cell culture supernatant was collected. The expression of the antigen proteins was detected by an ELISA assay using the D25 monoclonal antibody and palivizumab, respectively. The specific detection procedure was detailed as follows:
(1) the purified detection antibodies (D25 monoclonal antibody and palivizumab) obtained in Example 2 were respectively diluted to 1 µg/mL with ELISA coating buffer (Solarbio, C1050) and then added to the wells of a 96-well ELISA plate (Corning, 3590) at a volume of 100 µL per well; the plate was placed at 4 °C for 12 hours;
(2) the coating buffer was decanted, and the plate was washed once with PBS; subsequently, 200 µL of a 5% skim milk solution prepared in PBS was added to each well of the 96-well plate as a blocking solution, and the plate was then incubated at room temperature for 1 hour for blocking; after that, the plate was washed once with PBS;
(3) during the blocking process in step (2), the cell culture supernatant was diluted with a blocking solution; starting from a 5-fold dilution, the supernatant was serially diluted in a 3-fold gradient; then 100 µL of the culture supernatant stock of each RSV-A recombinant F protein to be tested or the diluted cell culture supernatant of partial of the RSV-A recombinant F proteins (RSV-A-Fm 28, 30, 32, 33, 34, 53) at various dilutions was added to each well of the ELISA plate, and the blocking solution alone was added to a well as a negative control; the plate was incubated at 37 °C for 2 hours, and then washed 4 times with PBST;
(4) the HRP-labeled Anti-His antibody (purchased from MBL) was added, and the plate was incubated at 37 °C for 1.5 hours, then washed 5-6 times with PBST; subsequently, TMB chromogenic solution was added for color development, after an appropriate reaction time, 2 M hydrochloric acid was added to terminate the reaction, and the OD₄₅₀ value was measured on a microplate reader.

The expression results of the respective RSV-A recombinant F proteins in the supernatant stocks were shown in Figures 1 and 2. As can be seen from these figures, compared to the negative control (NC) and the wild-type RSV-A F protein, the expression levels of all the detected RSV-A-Fm recombinant proteins were significantly improved; moreover, all the detected RSV-A-Fm recombinant proteins could bind to both palivizumab (which can recognize both pre-fusion and post-fusion conformations of F proteins) and the D25 monoclonal antibody (which specifically recognizes the pre-fusion conformation of F proteins), indicating that the RSV-A-Fm recombinant proteins of the present application were in the pre-fusion conformation.

The expression results of the RSV-A recombinant F proteins RSV-A-Fm 28, 30, 32, 33, 34, and 53 in the serially diluted supernatants were shown in Figures 3 and 4. As can be seen from these figures, even when the cell expression supernatants of RSV-A-Fm 28, 30, 32, 33, 34, and 53 were diluted 1000-fold, both the D25 monoclonal antibody and palivizumab were still able to detect the proteins, indicating that RSV-A-Fm 28, 30, 32, 33, 34, and 53 exhibited relatively high expression levels.

### Example 4: Expression, purification, and molecular weight identification of RSV-A-Fm recombinant proteins

In this example, HEK293T cells were transfected with the expression plasmids for the recombinant proteins RSV-A-Fm 30, 33, 34, 53 as constructed in Example 1 to express the corresponding recombinant proteins. The resulting proteins were purified by His affinity chromatography and gel filtration chromatography (also known as "molecular sieve chromatography"), and the proteins of interest were collected and subjected to analytical ultracentrifugation to determine the molecular weights thereof.

Specifically, HEK293T cells were cultured in DMEM medium supplemented with 10% FBS and then transfected with the expression plasmids for the recombinant proteins RSV-A-Fm 30, 33, 34, 53 as constructed in Example 1, respectively, when the cell density reached over 70%. After 4 - 6 hours of transfection, the cell culture medium was replaced with serum-free DMEM, and the cells were cultured for an additional 3 days. Then, the cell culture supernatant was collected. Subsequently, fresh DMEM was added to the cells, which were cultured for an additional 4 days, and then the cell culture supernatant was collected again. The cell culture supernatants from both collections were pooled and centrifuged at 5000 rpm for 30 minutes. The supernatant was filtered through a 0.22 µm filter membrane and loaded onto a HisTrap excel column (5 mL, GE Healthcare) to allow the binding of the proteins of interest. Non-specifically bound proteins were eluted with an elution buffer containing 20 mM Tris, 150 mM NaCl at pH 8.0, and 30 mM imidazole, and then the proteins of interest were eluted with an elution buffer containing 20 mM Tris, 150 mM NaCl at pH 8.0, and 400 mM imidazole. The fractions containing the proteins of interest were collected, concentrated, and subjected to a purification by molecular sieve chromatography (Superdex 200 Increase 10/300 GL or Superdex 200 Hiload 16/60 GE Healthcare) to obtain purified RSV-A-Fm recombinant protein antigens. At the same time, eluates corresponding to the peaks of the proteins of interest were collected for analytical ultracentrifugation to determine the molecular weights of the expressed recombinant proteins.

The UV absorption profiles from the molecular sieve chromatography were shown in the left panels of Figure 5, which showed that the elution profiles of the four recombinant proteins RSV-A-Fm 30, 33, 34, and 53 each displayed a single peak, with minimal or no impurity peaks, indicating that these recombinant proteins formed no or only a very small amount of aggregates. The eluates corresponding to the peaks of the single target proteins were collected for analytical ultracentrifugation, and the results were shown in the right panels of Figure 5, which showed that the molecular weights of RSV-A-Fm 30, 33, 34, and 53 were measured to be 168 kDa, 173 kDa, 181 kDa, and 175 kDa, respectively, which were consistent with the theoretical molecular weight of a trimer, indicating that the above RSV-A-Fm 30, 33, 34, and 53 proteins all existed as trimers.

### Example 5: Immunizing mice with RSV-A-Fm recombinant proteins

In this example, the recombinant proteins RSV-A-Fm 30, 33, 34, and 53 obtained in Example 4 were used to immunize mice, respectively. The experimental mice were BALB/c mice aged 4-6 weeks with an average weight of 15-20 g.

Specifically, mice were immunized with the recombinant proteins RSV-A-Fm 30, 33, 34, and 53 obtained in Example 4, respectively, and each of those recombinant proteins was divided into two groups: a pure protein immunization group and a protein-plus-adjuvant immunization group, in which the adjuvant used was AddaVax, a type of MF59 adjuvant. The immunization grouping as well as immunogens, immunogen doses, and adjuvants used for each group were shown in Table 1, in which blank boxes indicated "none". Each recombinant RSV-A-Fm protein was diluted in normal saline to the required concentration, and for the protein-plus-adjuvant immunization groups, the proteins were emulsified with the adjuvant prior to immunization. Each group consisted of 6 BALB/c mice aged 4-6 weeks with an average weight of 15-20g.

**Table 1**

| Group | Immunogen | Dose | Adjuvant |
|---|---|---|---|
| 1 | Fm33 | 5 µg | AddaVax |
| 2 | Fm33 | 20 µg | AddaVax |
| 3 | Fm33 | 20 µg | |
| 4 | Fm34 | 5 µg | AddaVax |
| 5 | Fm34 | 20 µg | AddaVax |
| 6 | Fm34 | 20 µg | |
| 7 | Fm53 | 5 µg | AddaVax |
| 8 | Fm53 | 20 µg | AddaVax |
| 9 | Fm53 | 20 µg | |
| 10 | Fm30 | 5 µg | AddaVax |
| 11 | Fm30 | 20 µg | AddaVax |
| 12 | Fm30 | 20 µg | |
| 13 | Normal saline | | |
| 14 | Normal saline | | AddaVax |

Mice in each group were intramuscularly injected with the recombinant protein vaccine or normal saline on days 0 and 14, with each immunization volume being 100 µL. Blood samples were collected from the tail vein on days 13 and 28, respectively. After allowing the blood to clot, the serum was separated by centrifugation at 3000 rpm for 10 minutes. The serum was then inactivated by incubation at 56 °C for 30 minutes and stored in a refrigerator at -80 °C.

### Example 6: Detection of vaccine-induced specific antibody titers by ELISA assay

In this example, the titers of specific IgG antibodies in the sera of mice immunized with the recombinant proteins RSV-A-Fm 30, 33, 34, and 53 as recited in Example 5 were detected by an ELISA assay.

Specifically, the procedure was conducted as follows:
(1) the recombinant proteins RSV-A-Fm 30, 33, 34, and 53 obtained in Example 4 were respectively diluted to 3 µg/mL with ELISA coating buffer (Solarbio, C1050) and then were added to the wells of a 96-well ELISA plate (Corning, 3590) at a volume of 100 µL per well; the plate was placed at 4 °C for 12 hours;
(2) the coating buffer was decanted, and the plate was washed once with PBS; subsequently, 100 µL of a 5% skim milk solution prepared in PBS was added to each well of the 96-well plate as a blocking solution, and the plate was then incubated at room temperature for 1 hour for blocking; after that, the plate was washed once with PBS;
(3) during the blocking process in step (2), the mouse serum sample was diluted with a blocking solution; starting from a 10-fold dilution, the serum sample was serially diluted in a 2-fold gradient; then 100 µL of the diluted serum solution was added to each well of the ELISA plate, and the blocking solution alone was added to a well as a negative control; the plate was incubated at 37 °C for 2 hours, and then washed 4 times with PBST;
(4) the goat anti-mouse secondary antibody conjugated with HRP (Abcam, ab6789) diluted 1:2000 in the blocking solution was added to the wells; the plate was incubated at 37°C for 1 hour and then washed 5-6 times with PBST; subsequently, TMB chromogenic solution was added for color development, after an appropriate reaction time, 2 M hydrochloric acid was added to terminate the reaction, and the OD₄₅₀ value was measured on a microplate reader.

The antibody titer was defined as the highest dilution of serum at which the reaction value was greater than 2.1 times the negative control value. When the reaction value of a sample at the lowest dilution (limit of detection) was still less than 2.1 times the background value, the titer of this sample was defined as half of the lowest dilution, i.e., 1:5.

The results were shown in Figure 6, which indicated that the four recombinant proteins RSV-A-Fm 30, 33, 34, and 53 all exhibited strong immunogenicity. Among others, after booster immunization with the recombinant proteins without adjuvant, the antigen-specific antibody titers induced by the four proteins all reached more than 10,000, and the incorporation of the AddaVax adjuvant further increased the antibody titers by 10-100 times.

### Example 7: Resolution of the crystal structure of RSV-A-Fm 30

According to the procedure described in Example 4, the RSV-A-Fm 30 protein was expressed and purified. For the purification, the eluate corresponding to the peak of the protein of interest was collected. The purified protein was concentrated to 10 mg/mL and mixed with the crystallization reservoir solution in a volume ratio of 1:1, and then the protein crystals were screened by Mosquito ^{®} Protein Crystallization Screening Liquid Workstation (TTP LabTech). The crystals were grown at 18 °C for diffraction analysis. The crystals were collected at Shanghai Synchrotron Radiation Center (SSRF), and diffraction data with a resolution of approximately 3 Å were obtained. The data were processed using HKL2000 software, and the structure of RSV-A-Fm30 was resolved by the molecular replacement method using the structure of DS-Cav2 (PDB: 5K6I) as a template.

The resolved structure of the RSV-A-Fm 30 was shown in Figure 7. As seen from Figure 7, RSV-A-Fm 30 was a F protein with the pre-fusion conformation.

### Example 8: Expression, isolation, and purification of detection antibodies

In this example, according to the methods for constructing antibody heavy chain and light chain sequences and their expression plasmids disclosed in the literatures (Jones HG., et al. PLoS Pathog 15(7): e1007944 (2019); Harshbarger, W., et al. Mabs 13(1): 1955812 (2021); Wen, X., et al. Nat Microbiol 2, 16272 (2017); Fabian Sesterhenn., et al. Science 368, eaay5051 (2020); Mousa, J., et al. Nat Microbiol 2, 16271 (2017)), expression plasmids for the heavy and light chains of the detection antibodies against RSV F protein, namely AM22, AM14, MPE8, 101F, and hRSV90 monoclonal antibodies, were constructed, respectively.

The specific procedures of antibody expression and isolation and purification followed those described in Example 2. The obtained antibody solution can be used directly or stored in a refrigerator at -80 °C for later use.

### Example 9: Expression and conformational characterization of RSV-A-Fm recombinant proteins

In this example, HEK293T cells were transfected with the expression plasmids for all the RSV-A-Fm recombinant proteins (Fm 1-76) as constructed in Example 1 to express the corresponding RSV-A-Fm recombinant proteins. Then, the expressed recombinant proteins were detected by ELISA using the detection antibodies prepared in Examples 2 and 8 for their expression levels and conformations. The specific procedure followed that described in Example 3.

The expression results of the respective RSV-A recombinant F proteins in the supernatant stocks were shown in Figures 8-15. As can be seen from these figures, compared to the wild-type RSV-A F protein, the expression levels of almost all the recombinant F proteins were significantly improved, with the exception of RSV-A-Fm2, Fm25, and Fm75, which showed only a slight increase; moreover, all the detected RSV-A-Fm recombinant proteins could bind not only to the monoclonal antibodies targeting epitope II which is common to both the pre-fusion and post-fusion conformations (e.g., palivizumab) and epitope IV which is common to both the pre-fusion and post-fusion conformations (e.g., 101F monoclonal antibody), but also to the monoclonal antibodies targeting the epitopes specific to the pre-fusion conformation, such as epitope Ø (e.g., D25 and AM22 monoclonal antibodies), the trimer-dependent spatial conformation epitope (e.g., AM14 monoclonal antibody), epitope V (e.g., hRSV90 monoclonal antibody), and epitope III (e.g., MPE8 monoclonal antibody), indicating that the RSV-A-Fm recombinant proteins of the present application were in the pre-fusion conformation.

### Example 10: Expression, purification, and SDS-PAGE identification of RSV-A-Fm recombinant proteins

In this example, HEK293T cells were transfected with the expression plasmids for the recombinant proteins RSV-A-Fm 29, 32, 38, 39, 40, 41, 46, 47, 48, 50, 51, *55,* 60, 63, 64, 67, 69, 70, 71, 72 as constructed in Example 1 to express the corresponding recombinant proteins. The resulting proteins were purified by His affinity chromatography and gel filtration chromatography (also known as "molecular sieve chromatography"), and the specific operation steps followed those recited in Example 4. After gel filtration chromatography, only a single peak was observed for each recombinant F protein, with minimal or no impurity peaks, indicating that these recombinant proteins formed no or only a very small amount of aggregates.

The eluates corresponding to the peaks of the protein of interest were collected for SDS-PAGE. The results were shown in Figure 16, which showed that for the recombinant proteins RSV-A-Fm 29, 32, 38, 39, 40, 41, 46, 47, 48, 50, 51, 55, 60, 63, 64, 67, 69, 70, 71, and 72, one distinct protein band was observed, indicating that they all had high purity; in addition, the molecular weights of different recombinant F proteins were slightly different, wherein the molecular weights of RSV-A-Fm 29, 32, 38, 39, 40, 41, 46, 47, 48, 50, 51, 55, 63 and 64 were in a range between 55 and 72 KDa, while those of RSV-A-Fm 60, 67, 69, 70, 71 and 72 were slightly smaller.

In addition, since each RSV-A-Fm recombinant F protein was a recombinant protein with a His-tag, Western Blot identification was further performed using an anti-His tag antibody conjugated to horseradish peroxidase in this example, and the results were shown in Figure 17. Figure 17 showed that the expression of His-tag was detected in each RSV-A-Fm recombinant F protein eluate, indicating that these RSV-A-Fm recombinant F proteins were all correctly expressed, as we expected.

### Example 11: Detection of RSV-A-Fm recombinant proteins for their stability

The recombinant proteins RSV-A-Fm 29, 30, 32, 33, 34, 38, 39, 40, 41, 46, 47, 48, 50, 51, 53, 55, 60, 63, 64, 67, 69, 70, 71, 72 obtained in Examples 4 and 10 were sterile filtered and stored at 30 °C. After 4 weeks, the proteins were detected by ELISA using an AM22 monoclonal antibody (targeting epitope Ø) and palivizumab (targeting epitope II), respectively, for the ability of binding to the antibodies so as to determine their long-term storage stability. The specific detection procedure was detailed as follows:
(1) the purified palivizumab and AM22 monoclonal antibodies obtained in Examples 2 and 8 were respectively diluted to 1 µg/mL with ELISA coating buffer (Solarbio, C1050) and then added to the wells of a 96-well ELISA plate (Corning, 3590) at a volume of 100 µL per well; the plate was placed 4 °C for 12 hours;
(2) the coating buffer was decanted, and the 96-well plate was washed once with PBS; subsequently, 200 µL of a 5% skim milk solution prepared in PBS was added to each well of the plate as a blocking solution, and the plate was then incubated at room temperature for 1 hour for blocking; after that, the plate was washed once with PBS;
(3) during the blocking process in step (2), the RSV-A-Fm recombinant proteins were diluted with a blocking solution; starting from an initial concentration of 300 ng/ml, each recombinant protein solution was serially diluted in a 3-fold gradient; then 100 µL of each serially diluted RSV-A-Fm recombinant protein solution was added to each well of the ELISA plate, and the blocking solution alone was added to a well as a negative control; the plate was incubated at 37 °C for 2 hours, and then washed 4 times with PBST;
(4) the HRP-labeled Anti-His antibody (purchased from MBL) was added, and the plate was incubated at 37°C for 1.5 hours and then washed 5-6 times with PBST; subsequently, TMB chromogenic solution was added for color development, after an appropriate reaction time, 2 M hydrochloric acid was added to terminate the reaction, and the OD₄₅₀ value was measured on a microplate reader.

The ELISA detection results using the AM22 monoclonal antibody and palivizumab were shown in Figures 18 and 19, respectively, which showed that the binding of each of the recombinant proteins RSV-A-Fm 29, 30, 32, 33, 34, 38, 39, 40, 41, 46, 47, 48, 50, 51, 53, 55, 60, 63, 64, 67, 69, 70, 71, and 72 to AM22 antibody or palivizumab did not change significantly after storage at 30 °C for 4 weeks compared to their respective initial proteins at week 0, indicating that these RSV-A-Fm recombinant proteins exhibited good stability.

### Example 12: Immunizing mice with RSV-A-Fm recombinant proteins

In this example, the recombinant proteins RSV-A-Fm 29, 30, 32, 33, 34, 38, 39, 40, 41, 46, 47, 48, 50, 51, 53*,* 55, 60, 63, 64, 67, 69, 70, 71, and 72 obtained in Examples 4 and 10 were used to immunize mice, respectively. The experimental mice were BALB/c mice aged 6-8 weeks with an average weight of 15-20 g.

Specifically, mice were immunized with the above RSV-A-Fm recombinant F proteins at a dose of 12 µg per immunization, respectively, where the adjuvant used was a composite adjuvant consisting of aluminum hydroxide (purchased from Croda) and CpG (purchased from Invivogen). The vaccine was prepared as follows: each RSV-A-Fm recombinant protein was diluted to the required concentration in normal saline, the aluminum hydroxide adjuvant was first mixed with the antigen protein, and then mixed with the CpG adjuvant. Normal saline was used as the placebo group. Each group consisted of four BALB/c mice aged 6-8 weeks with an average weight of 15-20 g.

Mice in each group were intramuscularly injected with the recombinant protein vaccine or normal saline on days 0 and 14, with each immunization volume being 100 µL. Blood samples were collected from the tail vein on days 13 and 28, respectively. After allowing the blood to clot, the serum was separated by centrifugation at 3000 rpm for 10 minutes. The serum was then inactivated by incubation at 56 °C for 30 minutes and stored in a refrigerator at -80 °C.

### Example 13: Detection of vaccine-induced antigen-specific antibody titers by ELISA assay

In this example, titers of specific IgG antibodies (expressed as Log values of antibody titers) in the sera of mice immunized with the recombinant protein vaccines of RSV-A-Fm 29, 30, 32, 33, 34, 38, 39, 40, 41, 46, 47, 48, 50, 51, 53, 55, 60, 63, 64, 67, 69, 70, 71 and 72 as recited in Example 12 were detected by an ELISA assay. For the specific procedure and the definition of antibody titer, please refer to Example 6.

The results were shown in Figure 20, which showed that the antigen-specific antibody titers induced by all the RSV-A-Fm recombinant F protein vaccines after the primary immunization reached more than 10,000, and could be further increased to more than 1,000,000 after the second immunization, indicating that all the RSV-A-Fm recombinant F proteins exhibited excellent immunogenicity.

### Example 14: Detection of vaccine-induced neutralizing antibody titers by trace neutralization assay

In this example, the neutralizing antibody titers in the sera of mice immunized with the recombinant protein vaccines of RSV-A-Fm 29, 30, 32, 33, 34, 38, 39, 40, 41, 46, 47, 48, 50, 51, 53, 55, 60, 63, 64, 67, 69, 70, 71 and 72 as recited in Example 12 were detected by trace neutralization assay.

Specifically, Vero cells were seeded into a 96-well plate one day in advance and were subjected to the following procedure when the cell confluence reached 80-90%. The sera of mice undergoing primary and booster immunizations were diluted with DMEM medium containing 1% FBS and 1 ‰ antibiotics. Specifically, starting from a 100-fold dilution, each serum was serially diluted 8 times in a 2-fold gradient. 100 TCID₅₀ of respiratory syncytial virus (RSV) was mixed with an equal volume of the diluted immunized mouse serum, and the resulting mixture was incubated at 37 °C for 1 hour. The cell culture supernatant was discarded, and 100 µl of the serum-virus mixture was added. For the positive control, an equal amount of respiratory syncytial virus (RSV) solution was added, and for the negative control, an equal volume of cell culture medium without virus was added, in place of the serum-virus mixture. After incubation at 37 °C for 5 hours, 100 uL of 1% FBS DMEM (including double antibodies) was supplemented and the resulting mixture was incubated at 37 °C for 4 days. The supernatant was discarded, and the cells were washed once with PBS, followed by adding a methanol-ethanol mixture at a volume ratio of 1:1 at 100 µL per well for cell fixing at room temperature for 10 minutes. Then the fixing solution was discarded and the cells were washed once with PBS. A 5% skim milk solution was used to block at 200 µL per well at 37 °C for 30 minutes. Then the blocking solution was discarded, and palivizumab (10 µg/ml) diluted in a 5% skim milk solution was added at 100 µl per well, followed by incubating at 37°C for 1 hour. The cells were washed 3 times with PBST (PBS + 0.05% Tween20), and then a horseradish peroxidase-labeled goat anti-human IgG secondary antibody (purchased from Beyotime Biotechnology) was added, followed by incubating at 37°C for 1 hour. The cells were washed 3 times with PBST (PBS + 0.05% Tween20) again. TMB chromogenic solution was added at 50 µL per well and the plate was placed at room temperature. After reacting for 6 minutes, 50 µL of 2M sulfuric acid solution was added to stop the reaction. OD₄₅₀ values were read using a microplate reader. The mean absorbance of cell-only control wells was taken as the background value. The background value was subtracted from all experimental readings. For each sample well, the neutralization titer was calculated as follows: 100% - [(OD₄₅₀ value for the sample well/ OD₄₅₀ value for the virus-only well) × 100]. The neutralization titer NT₅₀ value for each serum sample was calculated through the four-parameter logistic curve fitting method.

The neutralizing titer detection results were as shown in Figure 21, which showed that the neutralizing antibody titers in the sera of mice immunized with all the recombinant F protein vaccine formulations were significantly increased, as compared with the placebo group, and notably the neutralizing antibody titers induced by the RSV-A-Fm 29, 30, 32, 33, 34, 38, 39, 40, 41, 46, 47, 48, 50, 51, 53, 55, 60, 63, 64, 67, 70, 71 recombinant F protein vaccines reached over 3000, indicating that all the RSV-A-Fm recombinant F protein vaccines exhibited good immunogenicity.

### Example 15: Evaluation of vaccine-induced immune protection against viral challenge

Four weeks after the second immunization, mice immunized with various vaccines from Example 12 were intranasally challenged with respiratory syncytial virus Long strain at a dose of 10 ^ 4 PFU/50 µL. On day 5 after the viral challenge, the mice were euthanized, and their lungs were aseptically harvested. The lungs from four mice per group were weighed and recorded, and homogenized in DMEM medium using a tissue homogenizer, followed by centrifugation at 5000 g/min for 10 minutes to obtain the lung tissue supernatant. Respiratory syncytial virus in the lung tissue supernatant samples was quantified by plaque assay, and finally the virus copy number per gram of lung tissue was calculated.

The plaque assay was performed as follows: BHK cells were seeded into a 12-well plate at a density of 1×10⁵ cells per well one day in advance; the tissue stock solution was initially diluted 10-fold by adding 50 µL of the lung tissue supernatant into 450 µL of DMEM medium and mixed thoroughly, followed by six additional 10-fold serial dilutions. The cells to be infected were washed twice with PBS and infected with the diluted lung tissue supernatant sample at 400 µL per well; after incubation at 37 °C for 2 hours, the cells were washed with PBS, and then an equal volume mixture of 2% sodium carboxymethyl cellulose and 2 × DMEM was added at 1 mL per well. The cells were incubated at 37 °C for 4 days. Then, the cells were washed once with PBS. Subsequently, methanol was added to fix the cells at room temperature for 10 minutes. After another wash with PBS, PBST containing 5% skim milk was added for blocking at 37 °C for 1 hour. Next, palivizumab diluted with 5% skim milk was added, followed by incubation at 37 °C for 1 hour. After that, the cells were washed three times with PBST (i.e. PBS buffer containing 0.05% Tween20), then the horseradish peroxidase-labeled goat anti-human IgG secondary antibody (purchased from Biyuntian Bio) was added, followed by incubation at 37 °C for 1 hour. Then, the cells were washed another three times with PBST (i.e. PBS buffer containing 0.05% Tween20). Finally, an AEC substrate (purchased from BD Biosciences) was added for reaction. Brown spots were identified as positive spots, and the PFU value of each sample was counted.

The results were shown in Figure 22. As demonstrated in Figure 22, a high titer of respiratory syncytial virus was detected in the lung tissue of placebo-immunized mice following viral challenge, whereas no virus was detected in all vaccine-immunized groups, indicating that all the vaccines could protect the body from respiratory syncytial virus infection after immunization, and have achieved very good protective effects.

### Example 16: Resolution of the crystal structure of RSV-A-Fm 47

The recombinant protein RSV-A-Fm 47 was concentrated to 10 mg/mL and mixed with the crystallization reservoir solution in a volume ratio of 1:1, and then the protein crystals were screened by Mosquito^{®} Protein Crystallization Screening Liquid Workstation (TTP LabTech). The crystals were grown at 18 °C for diffraction analysis. The crystals were collected at Shanghai Synchrotron Radiation Center (SSRF), giving diffraction data of about 2.6 Å. The data were processed using HKL2000 software, and the structure of RSV-A-Fm 47 was finally resolved by the molecular replacement method using the structure of DS-Cav2 (PDB: 5K6I) as a template.

The resolved structure of the RSV-A-Fm 47 was shown in Fig. 23, which showed that the RSV-A-Fm 47 protein was a F protein in the pre-fusion conformation.

Finally, it should be noted that the above embodiments are only used to illustrate the technical solutions of the present application but not to limit it; although the present application has been described in detail with reference to the foregoing embodiments, it will be understood by one of ordinary skill in the art that the technical solutions described in the foregoing embodiments can be modified or some technical features can be equivalently substituted; however, these modifications or substitutions do not make the essence of the corresponding technical solutions departing from the spirit and scope of the present invention.

### Industrial applicability

The RSV-A recombinant F protein of the present application comprises at least one epitope specific to the pre-fusion F protein, and can form a stable, pre-fusion conformation of F protein trimer. Furthermore, the RSV-A recombinant F protein of the present application can be expressed stably in a uniform form and with a significantly increased yield. The formed F protein trimer has strong immunogenicity and thus can stimulate the body to produce a high-level antibody titer, which is of great significance for the clinical treatment, prevention, and control of respiratory syncytial virus.

## Claims

1. A respiratory syncytial virus subtype A (RSV-A) recombinant F protein, wherein the RSV-A recombinant F protein comprises RSV-A-F₁ peptide fragment and RSV-A-F₂ peptide fragment, wherein the RSV-A-F₁ peptide fragment and/or RSV-A-F₂ peptide fragment comprise at least one mutation to proline compared to the corresponding peptide fragment of the wild-type RSV-A F protein.

2. The RSV-A recombinant F protein of claim 1, wherein the RSV-A-F₁ peptide fragment corresponds to the amino acid fragment at positions 26-99, 26-103, 26-108, or 26-97 in the amino acid sequence of the wild-type RSV-A F protein as set forth in SEQ ID NO: 1, the RSV-A-F₂ peptide fragment corresponds to the amino acid fragment at positions 136-513, 145-513, 138-513, or 137-513 in the amino acid sequence of the wild-type RSV-A F protein as set forth in SEQ ID NO: 1, and the RSV-A-F₁ peptide fragment and/or RSV-A-F₂ peptide fragment comprise one or more mutations to proline compared to the corresponding peptide fragment of the wild-type RSV-A F protein.

3. The RSV-A recombinant F protein of claim 2, wherein the one or more mutations to proline are mutations in the amino acid sequence of the wild-type RSV-A F protein as set forth in SEQ ID NO: 1 selected from the group consisting of:
K65P, N67P, D73P, L138P, G139P, L141P, E161P, Q210P, I214P, S215P, N216P, Q279P, and S377P;
preferably, the one or more mutations to proline are selected from the following mutations or combinations of mutations:
N67P;
L141P;
Q279P;
S377P;
N67P+L141P;
N67P+ Q279P;
N67P+ S377P;
L141P+Q279P;
L141P+S377P;
Q279P+S377P;
N67P+L141P+Q279P;
N67P+L141P+S377P;
N67P+Q279P+S377P;
L141P+Q279P+S377P;
N67P+L138P+G139P;
L138P+G139P+Q279P;
L138P+G139P +S377P;
N67P+L138P+G139P+Q279P;
N67P+L138P+G139P+S377P;
N67P+L141P+Q279P+S377P;
L138P+G139P+Q279P+S377P;
N67P+L138P+G139P+Q279P+S377P;
N67P+L138P+G139P+L141P+Q279P+S377P;
optionally, the N67P mutation in the above mutations or combinations of mutations may be replaced by the K65P mutation or D73P mutation;
optionally, the L141P mutation in the above mutations or combinations of mutations may be replaced by the L138P mutation or G139P mutation;
optionally, the above mutations or combinations of mutations further comprise one or more mutations selected from the group consisting of an S215P mutation, an E161P mutation, an I214P mutation, an N216P mutation, and a Q210P mutation.

4. The RSV-A recombinant F protein of any one of claims 1 to 3, wherein the RSV-A-F₁ peptide fragment and the RSV-A-F₂ peptide fragment are connected directly or by a linking bridge;
preferably, the linking bridge is selected from the group consisting of:
(i) a (GS)m linking bridge, wherein m = 1-5, preferably 1-3, optionally comprising one or more amino acid mutations;
(ii) a (GGGGS)n linking bridge, wherein n = 1-5, preferably 1-3, optionally comprising one or more mutations to proline;
(iii) the native linking sequence between the wild-type F₁ and F₂ peptide fragments, which is part of the F₀ protein itself;
(iv) a sequence derived from the linking bridge (iii) by mutating the furin cleavage site thereon;
further preferably, the linking bridge comprises or consists of an amino acid sequence selected from the group consisting of SEQ ID NOs: 2-10.

5. The RSV-A recombinant F protein of any one of claims 1 to 4, wherein the recombinant F protein comprises or consists of an amino acid sequence selected from the group consisting of SEQ ID NOs: 29-100, or an amino acid sequence that is derived from any one of the amino acid sequences as set forth in SEQ ID NOs: 29-100 by the substitution, deletion, or addition of one or several amino acids therein and has the same or substantially the same immunogenicity as the amino acid sequence it is derived from.

6. The RSV-A recombinant F protein of any one of claims 1 to 5, wherein the recombinant F protein further comprises a trimerization tag;
preferably, the trimerization tag is located at the C-terminus and has an amino acid sequence selected from the group consisting of SEQ ID NOs: 24-27.

7. The RSV-A recombinant F protein of any one of claims 1 to 6, wherein the recombinant F protein further comprises a signal peptide;
preferably, the signal peptide is located at the N-terminus and has an amino acid sequence selected from the group consisting of SEQ ID NOs: 11-23.

8. A polynucleotide, which encodes the RSV-A recombinant F protein of any one of claims 1 to 7.

9. The polynucleotide of claim 8, wherein the polynucleotide is a DNA molecule or an mRNA molecule;
preferably, the DNA molecule comprises or consists of a DNA sequence as set forth in one of SEQ ID NOs: 105-180;
preferably, the mRNA molecule comprises or consists of an RNA sequence corresponding to the DNA sequence as set forth in one of SEQ ID NOs: 105-180.

10. A nucleic acid construct, which comprises the polynucleotide of claim 8 or 9, and optionally at least one expression regulatory element operably linked to the polynucleotide.

11. An expression vector, which comprises the nucleic acid construct of claim 10.

12. A host cell into which the polynucleotide of claim 8 or 9, the nucleic acid construct of claim 10, or the expression vector of claim 11 is transformed or transfected;
optionally, the host cell is a mammalian cell, an insect cell, a yeast cell, or a bacterial cell;
further optionally, the mammalian cell is a 293T cell, a 293F cell, or a CHO cell;
further optionally, the bacterial cell is an *Escherichia coli* cell.

13. A respiratory syncytial virus subtype A (RSV-A) recombinant F protein trimer, which is formed by the polymerization of three RSV-A recombinant F proteins of any one of claims 1 to 7.

14. Use of the RSV-A recombinant F protein of any one of claims 1 to 7, the polynucleotide of claim 8 or 9, the nucleic acid construct of claim 10, the expression vector of claim 11, the host cell of claim 12, or the RSV-A recombinant F protein trimer of claim 13 for the preparation of a vaccine for the prevention and/or treatment of respiratory syncytial virus infections.

15. A vaccine or immunogenic composition, which comprises the RSV-A recombinant F protein of any one of claims 1 to 7, the polynucleotide of claim 8 or 9, the nucleic acid construct of claim 10, the expression vector of claim 11, the host cell of claim 12, or the RSV-A recombinant F protein trimer of claim 13, and a physiologically acceptable vehicle, adjuvant, excipient, carrier, and/or diluent.

16. The vaccine or immunogenic composition of claim 15, wherein the vaccine or immunogenic composition is a respiratory syncytial virus recombinant protein vaccine which comprises the RSV-A recombinant F protein of any one of claims 1 to 7 or the RSV-A recombinant F protein trimer of claim 13, and an adjuvant;
optionally, the adjuvant is one or more selected from the group consisting of an aluminum adjuvant, an MF59 adjuvant, an MF59-like adjuvant, and AS series-like adjuvants.

17. The vaccine or immunogenic composition of claim 15, the vaccine or immunogenic composition is a respiratory syncytial virus DNA vaccine which comprises:
(i) a eukaryotic expression vector; and
(ii) a DNA sequence encoding the RSV-A recombinant F protein of any one of claims 1 to 7, preferably a DNA sequence as set forth in one of SEQ ID NOs: 105-180, which is constructed into the eukaryotic expression vector;
preferably, the eukaryotic expression vector is selected from the group consisting of pGX0001, pYAX1, pCAGGS, and pcDNA series vectors.

18. The vaccine or immunogenic composition of claim 15, wherein the vaccine or immunogenic composition is a respiratory syncytial virus mRNA vaccine which comprises:
(I) an mRNA sequence encoding the RSV-A recombinant F protein of any one of claims 1 to 7, preferably an mRNA sequence corresponding to the DNA sequence as set forth in one of SEQ ID NOs: 105-180; and
(II) a lipid nanoparticle.

19. The vaccine or immunogenic composition of claim 15, wherein the vaccine or immunogenic composition is a respiratory viral syncytial virus vector vaccine which comprises:
(1) a viral backbone vector; and
(2) a DNA sequence encoding the RSV-A recombinant F protein of any one of claims 1 to 7, preferably a DNA sequence as set forth in one of SEQ ID NOs: 105-180, which is constructed into the viral backbone vector;
optionally, the viral backbone vector is one or more selected from the following viral vectors: an adenovirus vector, a poxvirus vector, an influenza virus vector, and an adeno-associated virus vector.

20. The vaccine or immunogenic composition of any one of claims 15 to 19, wherein the vaccine or immunogenic composition is in the form of a nasal spray, an oral formulation, a suppository, or a parenteral formulation.
preferably, the nasal spray is selected from the group consisting of an aerosol, a spray, and a powder inhalant;
preferably, the oral formulation is selected from the group consisting of a tablet, a powder, a pill, a pulvis, a granule, a fine granule, a soft/hard capsule, a film-coated preparation, a pellet, a sublingual tablet, and an ointment;
preferably, the parenteral formulation is a transdermal formulation, an ointment, a plaster, a topical liquid, or an injectable or bolus-injectable formulation.

21. A method for preparing the RSV-A recombinant F protein of any one of claims 1 to 5, wherein the method comprises:
adding a nucleotide sequence encoding a signal peptide to the 5' end of the codon-optimized nucleotide sequence encoding the RSV-A recombinant F protein of any one of claims 1 to 5, and adding nucleotide sequences encoding a trimerization tag and a His-tag, as well as a stop codon to the 3' end thereof, performing cloning and expression, screening correct recombinants, and then transfecting the correct recombinants into an expression system cell for expression, collecting the cell culture supernatant, and isolating the RSV-A recombinant F protein therefrom;
optionally, the expression system cell is a mammalian cell, an insect cell, a yeast cell, or a bacterial cell;
further optionally, the mammalian cell is a 293T cell, a 293F cell, or a CHO cell;
further optionally, the bacterial cell is an *Escherichia coli* cell.
